(19) Europäisches Patentamt / European Patent Office / Office européen des brevets

(11) **EP 2 975 392 B1**

(12) **EUROPEAN PATENT SPECIFICATION**

(45) Date of publication and mention
of the grant of the patent:
**05.09.2018 Bulletin 2018/36**

(21) Application number: **14765206.9**

(22) Date of filing: **13.03.2014**

(51) Int Cl.:
*G01N 27/447* [(2006.01)]   *G01N 27/62* [(2006.01)]
*G01N 33/36* [(2006.01)]   *G01N 33/561* [(2006.01)]
*G01N 33/68* [(2006.01)]

(86) International application number:
**PCT/JP2014/056636**

(87) International publication number:
**WO 2014/142229 (18.09.2014 Gazette 2014/38)**

(54) **DISCRIMINATION METHOD OF ANIMAL HAIR FIBER**

VERFAHREN ZUR TIERHAARFASERIDENTIFIKATION

PROCÉDÉ D'IDENTIFICATION DE FIBRES DE POILS D'ORIGINE ANIMALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2013 JP 2013052625**

(43) Date of publication of application:
**20.01.2016 Bulletin 2016/03**

(73) Proprietor: **Nissenken Quality Evaluation Center
Tokyo 111-0051 (JP)**

(72) Inventor: **MASUDA, Takeshi
Tsuruoka-shi, Yamagata 997-0017 (JP)**

(74) Representative: **Kalhammer, Georg et al
Lederer & Keller
Patentanwälte Partnerschaft mbB
Unsöldstrasse 2
80538 München (DE)**

(56) References cited:
EP-A1- 1 847 831    WO-A1-2013/154208
JP-A- 2003 204 798    JP-A- 2004 121 229

- MARSHALL ET AL: "Methods and Future
  Prospects for Forensic Identification of Hairs by
  Electrophoresis", JOURNAL - FORENSIC
  SCIENCE SOCIETY, FORENSIC SCIENCE
  SOCIETY, HARROGATE, GB, vol. 25, no. 1, 1
  January 1985 (1985-01-01), pages 57-66,
  XP022571068, ISSN: 0015-7368

- RANE PRIYANKA P ET AL: "Evaluating Protein
  Patterns of Speciality Fibres for Identification to
  Combat False Labeling", INTERNATIONAL
  JOURNAL OF ZOOLOGICAL RESEARCH,
  ACADEMIC JOURNALS, US, vol. 6, no. 4, 1
  October 2010 (2010-10-01) , pages 286-292,
  XP007917524, ISSN: 1811-9778, DOI:
  10.3923/IJZR.2010.286.292
- M.ZOCCOLA ET AL.: 'CROMATOGRAFIA
  LIQUIDA E GEL ELETTROFORESI' TINCTORIA
  vol. 93, no. 9, 1996, pages 57 - 62, XP008181011
- KIRSTEN KERKHOFF ET AL.: 'DNA analytical
  identification of animal hair fibers in textiles'
  MELLIAND TEXTILBERICHTE vol. 87, no. E83-E8,
  2006, pages 354 - 356, XP008181007
- 'Sen'i Sangyo ni Kakaru Heisei 21 Nendo 'Joho
  Gyomu' ni Okeru 'Cashmere Hyoji no Tekiseika
  ni Kakaru Chosa Jigyo' Itaku Seika Hokokusho'
  ORGANIZATION FOR SMALL & MEDIUM
  ENTERPRISES AND REGIONAL INNOVATION
  February 2010, JAPAN, XP008174289
- MASATOSHI (TAKAHAMA) ICHIDA: 'Kinu (Kasan
  to shite no Silk' GEKKAN SEN'I vol. 65, no. 2, 2012,
  pages 125 - 130, XP008181017
- KLAUS HOLLEMEYER ET AL.: 'Identification and
  Quantification of Feathers, Down, and Hair of
  Avian and Mammalian Origin Using Matrix-
  Assisted Laser Desorption/Ionization Time-of-
  Flight Mass Spectrometry' ANAL. CHEM. vol. 74,
  2002, pages 5960 - 5968, XP002593343

EP 2 975 392 B1

**Description**

TECHNICAL FIELD

The present invention relates to a discrimination method of animal hair fibers for discriminating a kind of an animal serving as the origin of the animal hair fibers, and for discriminating fibers in which the animal hair fibers having different origins are mixed, and further, for discriminating their mixing ratio.

BACKGROUND ART

In the market, fiber products using many animal hair fibers have been distributed as high-quality goods. Into the fiber products using cashmere which is particularly deemed to be high-quality goods among the animal hair fibers, camouflage has been frequently carried out by mixing the other animal hair fibers. Accordingly, when a general consumer purchases fiber products, the person cannot distinguish camouflage, which is a big problem for fair trading. For example, elaborate camouflage has been carried out by mixing hairs of yak (the genus Bos) which can be hardly distinguished from cashmere (the genus Capra), or mixing wool (the genus Ovis) by removing scale (which is called to "descaling".).

Thus, at the time of exporting and importing of the fiber products, discrimination is carried out in the inspection organizations relating to the fibers of the respective countries to maintain the safety of the transaction. In these inspection organizations, for example, in Japan, discrimination has been carried out based on JIS L 1030-1 (Testing methods for quantitative analysis of fibre mixtures-Part 1: Testing methods for fibre identification) and JIS L 1030-2 (Testing methods for quantitative analysis of fibre mixtures of textiles-Part 2: Testing methods for quantitative analysis of fibre mixtures).

In this discrimination method, for discriminating the animal hair fibers, the animal hair fibers which are an object for inspection are compared with standard photographic samples by an inspector using an optical microscope by visual observation. Also, in this discrimination method, for obtaining a mixing ratio of the animal hair fibers, it has been carried out by the method in which a number of yarns or a diameter of a different kind of the animal hair fibers contained in the animal hair fibers which are an object for inspection is obtained, or sorted and measured the weight of respective fibers, etc., by the inspector using an optical microscope by visual observation.

Accordingly, in these methods, there is a problem that fluctuation in the discrimination results occurs due to the difference in experience and know-how of the inspector of the respective inspection organizations. In addition, for obtaining the mixing ratio, there is a problem that an operation with extremely complicated for a long period of time by the inspector is accompanied. Moreover, when elaborate camouflage is made to the animal hair fibers, there is a problem that accurate discrimination cannot be carried out by the above-mentioned method alone.

To the contrary, it has been proposed a method to complement the above-mentioned discrimination method using an optical microscope with naked eyes. For example, in the following mentioned Patent Document 1, an identification method of the animal hair fibers by DNA has been proposed. According to this method, DNA are amplified by the polymerase chain reaction (PCR) using DNA extracted from the animal hair fibers sample and a primer specific to animal species, and the base sequences of the amplified components are analyzed to discriminate the animal species serving as the origin of the animal hair fibers sample.

Also, in the following mentioned Patent Document 2, it has been proposed a discrimination method of a mixing ratio of the animal hair fibers in the animal hair fiber product. According to this method, real time PCR is carried out by using a probe which specifically detect DNA extracted from the animal hair fibers sample, a primer specific to the animal species and a sequence amplified by the said primer to obtain a ratio of the relative values of the DNA amount derived from the respective animal species, and a mixing ratio of the animal hair fibers is discriminated based on a previously prepared correspondence table with a ratio of the relative values of the DNA amount of a series of the standard mixed animal hair fibers.

Patent Document 3 describes a method for the recognition and the quantitative evaluation of mixed textile fibers of animal source.

Non-Patent Document 1 reports on a two-dimensional electrophoretic procedure for discriminating between hair samples of different species.

PRIOR ART DOCUMENTS

PATENT DOCUMENTS

Patent Document 1: JP 2000-210084A
Patent Document 2: JP 2004-121229A

Patent Document 3: EP 1847831 A1

NON-PATENT DOCUMENT

Non-Patent Document 1: Marshall RC et al. (1985) Journal of the Forensic Science Society, Volume 25, pages 57-66.

PROBLEMS TO BE SOLVED BY THE INVENTION

The identification method of the above-mentioned Patent Document 1, and the discrimination method of the mixing ratio in the above-mentioned Patent Document 2 are each to discriminate animal species serving as the origins of the animal hair fibers or a mixing ratio thereof by the gene sequence of DNA extracted from the animal hair fibers. These methods utilize the fact that gene sequences are different by each animal species, and it is expected to provide a biologically objective result.

However, the operations of extracting DNA from the animal hair fibers which are a sample for discrimination, and amplifying the same are extremely complicated, so that it is not easy to carry out the above as a regular work in many textile-related inspection organization. In addition, to the fiber products distributed to the market, various pre-treatments or dyeing processings have generally been carried out. These pre-treatments or dyeing processings have been carried out to the animal hair fibers by a physical or chemical method, which causes certain damages to the animal hair fibers themselves. Accordingly, it is difficult to extract DNA without damage from the animal hair fibers to which pre-treatments or dyeing processings have been carried out, whereby there is a problem that accurate discrimination cannot be carried out.

Thus, an object of the present invention is to provide a discrimination method of animal hair fibers, dealing with the above-mentioned problems, in which the discrimination operation is relatively simple and easy, and has objectivity, discrimination can be carried out without relying on experience and know-how of an inspector, and even when a pre-treatment or dyeing processing has been carried out to the animal hair fibers, accurate discrimination results can be obtained.

For solving the above-mentioned problems, the present inventors have intensively studied, and as a result, they have found that, by attracting attention to the proteins constituting the animal hair fibers, a combination of the group of proteins extracted from the animal hair fibers is different depending on the kinds of the original animals of the animal hair fibers. In addition, they have found that a combination of the peptide groups derived from a specific protein taken out from these groups of proteins is different depending on the kinds of the original animals of the animal hair fibers. From these findings, the present inventors have reached the completion of the present invention.

SUMMARY OF THE INVENTION

The present invention relates to subject matter defined in the following items (1) to (7):

(1) A discrimination method of animal hair fibers which comprises
an extraction process for extracting a group of proteins from animal hair fibers which constitute the animal hair fibers sample,
an electrophoretic process for obtaining an electrophoretic pattern by separating the extracted group of proteins with an electrophoretic means and staining the gel after the electrophoresis with a protein dyeing liquid, and
a first discrimination process for discriminating kinds of animals serving as the origins of the animal hair fibers sample by comparing the obtained electrophoretic pattern with previously prepared electrophoretic patterns of single animal hair fibers in which kinds of animals serving as the origin have been known,
wherein
a series of electrophoretic patterns of previously prepared mixed animal hair fibers in which the animal hair fibers different in kinds of animals serving as the origin have been mixed with a series of mixing ratios is obtained in the same manner as in the extraction process and the electrophoretic process, and the method further comprises
a second discrimination process for discriminating the animal hair fibers sample being originated from at least two kinds of animals, kinds of at least two kinds of animals serving as the origins of the animal hair fibers sample, and a mixing ratio of the animal hair fibers sample by comparing the electrophoretic pattern obtained from the animal hair fibers sample with a series of electrophoretic patterns obtained from the mixed animal hair fibers.

(2) The discrimination method of animal hair fibers according to item (1), wherein
the method has a process for accumulating as a database by analyzing a relation of an intensity and relative mobility of each band with regard to the electrophoretic pattern of the single animal hair fibers, and a series of electrophoretic patterns obtained from the mixed animal hair fibers,

a process for analyzing a relation between an intensity and a relative mobility of each band with regard to the electrophoretic patterns obtained from the animal hair fibers sample, and

a third discrimination process for discriminating the animal hair fibers sample being originated from at least two kinds of animals, kinds of at least two kinds of animals serving as the origins of the animal hair fibers sample, and a mixing ratio of the animal hair fibers sample by collating analytical data of the animal hair fibers sample with a data group of the database, with consistency of the analytical data and the data group of the database as an index.

(3) The discrimination method of animal hair fibers according to item (1) or (2), wherein

a mixing ratio of the animal hair fibers sample is discriminated by improving consistency of a mixing ratio of the animal hair fibers sample and a mixing ratio of the comparative animal hair fibers, by repeating at least once of each step of obtaining an electrophoretic pattern of comparative animal hair fibers in which using a mixing ratio of the animal hair fibers sample obtained in the second discrimination process or the third discrimination process, the single animal hair fibers in which the kinds of the original animals have been known are mixed with the same mixing ratio, and

each step of carrying out discrimination of the second discrimination process or the third discrimination process again to the electrophoretic pattern of the obtained comparative animal hair fibers.

(4) The discrimination method of animal hair fibers according to item (1), wherein

the mixed animal hair fibers are materials in which two kinds of the animal hair fibers in which the kinds of the original animals have been known are mixed with a series of mixing ratios, and the method further comprises

a process for obtaining an intensity of a band at a previously recognized relative mobility which is specific to the two kinds of the animal hair fibers with regard to a series of electrophoretic pattern obtained from the mixed animal hair fibers,

a process for obtaining an intensity of a band at a previously recognized relative mobility with regard to the electrophoretic pattern obtained from the animal hair fibers sample, and

a fourth discrimination process for discriminating a mixing ratio of the animal hair fibers sample by collating the intensity of the band at the previously recognized relative mobility of the animal hair fibers sample with the intensity of the band at the previously recognized relative mobility of the mixed animal hair fibers, with consistency of intensities of these bands as an index.

(5) The discrimination method of animal hair fibers according to item (4), wherein

the two kinds of the animal hair fibers are cashmere and wool.

(6) The discrimination method of animal hair fibers according to any one of items (1) to (5), wherein

in the electrophoretic process for obtaining an electrophoretic pattern,

an electrophoretic gel having resolution at a region of a molecular weight of 25 kDa or less is capable of separating 10 or more bands is used at least to the electrophoretic pattern of the single animal hair fibers originating from cashmere among the electrophoretic patterns of the single animal hair fibers in which the kinds of the original animals have been known.

(7) The discrimination method of animal hair fibers according to any one of items (1) to (6), wherein

in the extraction process for extracting the group of proteins,

a reducing agent is used for splitting a disulfide bond in a molecule or between molecules of the protein, and the reducing agent is 2-mercaptoethanol with a concentration of more than 6% in a concentration of % by volume, or dithiothreitol with 8 to 12 mM (millimole/liter) in a molar concentration.

EFFECTS OF THE INVENTION

According to the above-mentioned constitution, the present invention has an extraction process and an electrophoretic process, a group of proteins is extracted from the animal hair fibers sample, and an electrophoretic pattern obtained from the group of proteins is compared with electrophoretic patterns of the single animal hair fibers in which the kinds of the original animals have been already known. This is based on the fact that a combination of a group of proteins constituting the animal hair fibers is different depending on the kinds of the animals.

Accordingly, objective discrimination can be carried out, and the kinds of the original animals of the animal hair fibers sample can be accurately discriminated. These operations are relatively simple and easy, and it is an instrumental analysis, so that no fluctuation in the discrimination results due to the difference in experience and know-how of an inspector occurs.

Also, even when camouflage such as descaling, etc., has been done to the animal hair fibers, in the present invention, proteins are objective materials, so that no difficulty is accompanied in the extraction as in the conventional extraction of DNA due to damage by camouflage.

Further, according to the above-mentioned constitution, the electrophoretic pattern of the animal hair fibers sample is compared with the electrophoretic patterns of the mixed animal hair fibers in which the animal hair fibers having different kinds of the animals serving as the origin are mixed with a series of mixing ratios. According to this procedure, even when the animal hair fibers sample are mixed animal hair fibers originated from at least two kinds of animals, the kinds of at least two kinds of animals serving as the origin and the mixing ratio thereof can be discriminated.

Further, according to the above-mentioned constitution, by analyzing a series of electrophoretic patterns obtained from the single animal hair fibers and the mixed animal hair fibers in which the kinds of the original animals have been already known, and making these as database, further accurate discrimination can be carried out. According to this procedure, more accurate discrimination can be carried out relatively simple and easy, and objectively.

Moreover, according to the above-mentioned constitution, by using the mixing ratio of the animal hair fibers sample obtained in the second discrimination process or the third discrimination process, the comparative animal hair fibers with the same mixing ratio are prepared. An electrophoretic pattern of the comparative animal hair fibers is obtained, and when it is substantially matched with the electrophoretic pattern of the animal hair fibers sample, accuracy of the firstly obtained mixing ratio of the animal hair fibers sample is confirmed.

On the other hand, when the electrophoretic pattern of the obtained comparative animal hair fibers is markedly different from the electrophoretic pattern of the animal hair fibers sample, the above-mentioned operation is again repeated to the comparative animal hair fibers in which the mixing ratio has been adjusted. According to this procedure, accuracy of the mixing ratio of the animal hair fibers sample can be improved, and more accurate discrimination can be carried out relatively simple and easy, and objectively.

Also, according to the above-mentioned constitution, when the animal hair fibers sample is constituted by the specific two kinds of the animal hair fibers, by collating a relation of relative mobility and an intensity of the band which are specific to these two kinds of the animal hair fibers, a mixing ratio of the animal hair fibers sample can be discriminated. In this case, the specific two kinds of the animal hair fibers may be cashmere and wool.

Further, according to the above-mentioned constitution, an electrophoretic gel to be used for the electrophoretic process may be a gel which has a resolution at the region of a molecular weight of 25 kDa or less that is separatable to 10 or more bands with regard to the electrophoretic pattern of cashmere. According to this procedure, the electrophoretic pattern of the animal hair fibers sample becomes clear so that more accurate discrimination can be carried out.

Moreover, according to the above-mentioned constitution, as a reducing agent to be used for splitting a disulfide bond of the protein in the extraction process, 2-mercaptoethanol with a concentration of more than or 6% in a concentration of % by volume, or 8 to 12 mM (millimole/liter) of dithiothreitol in a molar concentration may be used. According to this procedure, extraction efficiency of the protein from the animal hair fibers sample is improved, and the electrophoretic pattern becomes clear so that more accurate discrimination can be carried out.

Accordingly, in the present invention, it can be provided a discrimination method of the animal hair fibers in which the discrimination operation is relatively simple and easy, and has objectivity, discrimination can be carried out without relying on experience and know-how of an inspector, and even when a pre-treatment or dyeing processing has been done to the animal hair fibers, accurate discrimination result can be obtained.

BRIEF DESCRIPTION OF THE DRAWINGS

Fig. 1 is gel images showing electrophoretic patterns of the animal hair fibers of cashmere (the genus Capra), camel (the genus Camelidae), wool (the genus Ovis) and yak (the genus Bos) in the first embodiment according to the present invention;

Fig. 2 is an analyzed chart showing the relation between the relative mobility and the band intensity by analyzing the gel images of cashmere (the genus Capra) and yak (the genus Bos) among the electrophoretic patterns of Fig. 1;

Fig. 3 is an analyzed chart showing the relation between the relative mobility and the band intensity by analyzing the gel images of cashmere (the genus Capra) and wool (the genus Ovis) among the electrophoretic patterns of Fig. 1;

Fig. 4 is gel images showing a series of electrophoretic patterns in which the animal hair fibers of cashmere (the genus Capra) and yak (the genus Bos) mixed with different mixing ratios in the first embodiment according to the present invention;

Fig. 5 is a graph visualized a part of the database prepared from electrophoretic patterns of a plural number of standard samples in the second embodiment according to the present invention;

Fig. 6 is gel images showing electrophoretic patterns obtained by using two kinds of gels having different resolution to the animal hair fibers of cashmere (the genus Capra), wool (the genus Ovis) and yak (the genus Bos) in the third embodiment according to the present invention;

Fig. 7 is gel images comparing electrophoretic patterns obtained by using two kinds of gels having different resolution to cashmere (the genus Capra) among the electrophoretic patterns of Fig. 6;

Fig. 8 is gel images comparing electrophoretic patterns to yak (the genus Bos) when a concentration or a kind of the reducing agent in the extracting liquid has been changed in the third embodiment according to the present invention;

Fig. 9 is gel images showing change in electrophoretic patterns to two kinds of the animal hair fibers sample in "checking test" (test for confirming calculated result) in the third embodiment according to the present invention;

Fig. 10 is gel images showing electrophoretic patterns of the standard samples in which a mixing ratio of cashmere (the genus Capra) and wool (the genus Ovis) has been changed and the animal hair fibers sample in the fourth embodiment according to the present invention;

Fig. 11 is an analyzed chart which is analyzed the gel images of the standard samples and the animal hair fibers sample in Fig. 10;

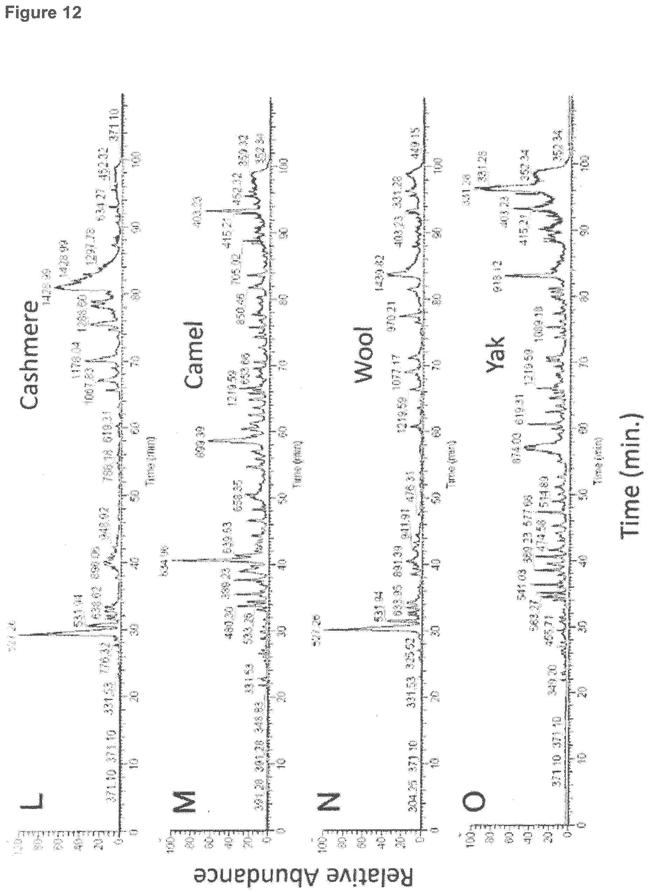

Fig. 12 is a graph showing mass spectrometric patterns of the animal hair fibers of cashmere (the genus Capra), camel (the genus Camelidae), wool (the genus Ovis) and yak (the genus Bos) in the first further aspect of the disclosure;

Fig. 13 is a graph visualized a part of the database prepared from mass spectrometric patterns of a plural number of standard samples in the third further aspect of the disclosure;

Fig. 14 is a gel image showing an electrophoretic pattern of the animal hair fibers sample (cashmere) and an analyzed chart in Example 1;

Fig. 15 is a gel image showing an electrophoretic pattern of the animal hair fibers sample (yak) and an analyzed chart in Example 1;

Fig. 16 is a gel image showing an electrophoretic pattern of the animal hair fibers sample (a mixture of cashmere and yak) and an analyzed chart in Example 1;

Fig. 17 is a graph showing a mass spectrometric pattern of the animal hair fibers sample (cashmere) in Example 3;

Fig. 18 is a graph showing a mass spectrometric pattern of the animal hair fibers sample (yak) in Example 3; and

Fig. 19 is a graph showing a mass spectrometric pattern of the animal hair fibers sample (a mixture of cashmere and yak) in Example 3.

EMBODIMENTS OF THE INVENTION

In the present invention, the animal hair fibers include all the hair fibers comprising a natural keratin material as a main component. Accordingly, it includes wool of sheep (it is referred to as "wool" in the present invention.), and there may be mentioned animal hair other than sheep, for example, cashmere goat hair "cashmere", angora goat hair "mohair", angora rabbit hair "angora", a kind of cattle: yak hair "yak", camel hair "camel", small humpless camel: alpaca hair "alpaca", similar small humpless camel: vicuna hair "vicuna", similar small humpless camel: llama hair "llama", etc.

Also, in the animal hair fibers according to the present invention, other than the above, those which are used as peltry or fur are also included. For example, there may be mentioned "fox" which is fur of fox, a kind of Japanese minks: mink hair "mink", a kind of mouse: chinchilla hair "chinchilla", rabbit hair "rabbit", etc. As can be understood from these examples, in the present invention, the same name is used in the name of an animal and the name of an animal hair.

In the present invention, the fiber product using the animal hair fibers may be mentioned, for example, woven fabric or knitted fabric using yarn which has spun the animal hair fibers or nonwoven fabric such as felt, etc. However, the invention is not limited by these, peltry or fur, etc., are included, and further, the case where it is used as stuffing, etc., in the hair fibers as such is also included. In addition, in these fiber products, those which have been subjected to a dyeing processing or various kinds of finishing processings are included. In particular, in the present invention, the case where a fiber product in which other inexpensive animal hair fibers are mixed with high class animal hair fibers such as cashmere, etc., and a fiber product in which animal hair fibers camouflaged by various kinds of processings have been mixed are discriminated is also an object of the same.

Also, in the present invention, the kinds of the animals do not mean "species" alone as a unit of classification in biology, but are to be used in a wide sense back to "the genus" or "the family". Accordingly, the kind of the animals to be discriminated in the discrimination method of the animal hair fibers according to the present invention means not only to discriminate the "species" of the animal which are origins of the animal hair fibers but also to discriminate "the genus" of the animals serving as the origin, or to discriminate "the family" of the animals serving as the origin in some cases.

In the following, the discrimination method of the animal hair fibers according to the present invention is explained in detail by the respective embodiments.

<<First embodiment>>

The discrimination method of the animal hair fibers according to this first embodiment has an extraction process, an electrophoretic process, a first discrimination process and a second discrimination process. The method is as specified in independent claim 1. In this first embodiment, first, a group of proteins constituting the animal hair fibers is extracted (extraction process) from the animal hair fibers which is an inspection object (in the following, it is referred to as "the animal hair fibers sample".). Next, the extracted group of proteins was separated by an electrophoresis apparatus to obtain an electrophoretic pattern (protein pattern) due to the difference of the molecular weights of the respective proteins (electrophoretic process).

Incidentally, gene sequences are different with the kinds of the animals, and the proteins are encoded in the gene. Accordingly, amino acid sequences of the proteins are also different with the kinds of the animals, so that the electrophoretic pattern of the group of proteins extracted from the animal hair fibers is specific to the kinds of animals. Based on this fact as a basis, the obtained electrophoretic pattern of the animal hair fibers sample is compared with the electrophoretic patterns of the several kinds of the single animal hair fibers the kinds of the animals serving as the origin have been known, the kind of the animal serving as the origin of the animal hair fibers sample is discriminated (the first discrimination process).

In the following, the respective processes in this first embodiment are explained in detail.

(1-1) Extraction process

The main constitutional proteins of the animal hair fibers are keratin and keratin-related protein, and in these proteins, there exist many kinds of molecular species having similar structures. These groups of proteins are different in their combination depending on the kinds of the animal. In the present invention, the constitution of these groups of proteins, i.e., a combination of a plural number of proteins is analyzed, whereby a kind of the animal serving as the origin of the animal hair fibers is discriminated.

In the present invention, a group of proteins is extracted from the animal hair fibers which constitute the same. Thus, by paying attention to the group of proteins which constitute the animal hair fibers, even when a camouflage processing such as descaling, etc., has been done on the surface of the animal hair fibers, it is possible to extract proteins so long as the animal hair itself remains. In this point, it contrasts with the case where extraction of DNA from the animal hair fibers to which a camouflage processing has been done is extremely difficult.

The present inventors have investigated electrophoretic patterns by extracting a group of proteins with regard to raw fiber itself, crushed raw fiber, bleached animal hair and descaled animal hair of the animal hair fibers, in the same manner. As a result, it was confirmed that there were not so remarkable change in these electrophoretic patterns. Accordingly, in the present invention, the proteins may be extracted after crushing the animal hair fibers sample, or the proteins may be extracted while maintaining the shape of the animal hair without crushing. However, with regard to the descaled animal hair fibers, a degree of the descaling is unknown so that the proteins are preferably extracted after crushing the animal hair fibers sample with a certain extent.

The specific extraction operation of the proteins is carried out as follows. First, when dyeing, etc., has been done to the animal hair fibers sample, the electrophoretic pattern is sometimes disturbed. In the present invention, it is preferred to carry out removal operations of a dye, a finishing agent and an auxiliary agent, etc., before extraction of the proteins. According to this procedure, precision of the electrophoretic pattern is raised and accurate discrimination can be done without being affected by a dye, etc., in the subsequent electrophoretic process.

In this washing operation, so as not to extract the proteins, it is preferred to employ a method in which, for example, washing is carried out by using a washing solution, etc., containing deoxycholic acid and lauroyl sarcosinic acid, at a temperature of 70°C to 100°C, preferably at 90°C to 95°C for several times. Also, with regard to the dyed animal hair fibers sample, a decoloration treatment may be carried out in accordance with the kind of the dye. For example, there may be mentioned an oxidation treatment, a reduction treatment, an acid treatment, an alkali treatment or a chelating treatment, etc. In these cases, it is preferably carried out under the conditions that the proteins are not broken and not extracted.

Next, an extraction operation of the proteins is carried out. As the extracting liquid of the proteins, an aqueous solution containing various kinds of surfactants, etc., may be generally used. The surfactant to be used is not particularly limited so long as it can solubilize the proteins without damaging the same and, for example, sodium dodecyl sulfate (SDS) is preferably used as a surfactant for heightening solubility of a keratin protein.

In addition, at the time of extraction of the proteins, solubilization becomes easy by reducing a disulfide bond (S-S bond) in the proteins to split the bond to make it as a cysteine residue. Therefore, in the extracted liquid, a reducing agent is preferably used in combination. These reducing agents may be mentioned, for example, 2-mercaptoethanol (2-ME), dithiothreitol (DTT), tris(2-carboxyethyl)phosphine (TCEP), etc.

In this extraction operation, the operations that the animal hair fibers sample is dipped in an extracting liquid using both of a surfactant and a reducing agent, extracted at a temperature of 70°C to 100°C, preferably 90°C to 95°C or so and the extracted liquid is recovered are repeated several times. After recovering these extracted liquids, the liquids are concentrated to a predetermined concentration and used in the subsequent electrophoretic process.

Here, it is preferred to block the cysteine residue so as not to form a disulfide bond again by the cysteine residue of the protein. For blocking the cysteine residue, a general method may be used and may be mentioned, for example, a method of alkylating using iodoacetamide, iodoacetic acid, acrylamide, etc. Incidentally, it is preferred that the total amount of the protein thus obtained is quantitatively determined.

(1-2) Electrophoretic process

Next, constituting proteins of the group of proteins obtained in the extraction process are separated by using the electrophoresis method. The electrophoresis method to be used is not particularly limited, and may be any method. In the present invention, a polyacrylamide gel electrophoresis method (SDS-PAGE method) using sodium dodecyl sulfate (SDS) is preferably used.

In general, the polyacrylamide gel used in the SDS-PAGE method has a three-dimensional structure by copolymerization of the acrylamide and N,N'-methylenebisacrylamide which is a crosslinking agent thereof. The sum of the acrylamide and the N,N'-methylenebisacrylamide is called as a gel concentration, and a gel with 10% to 20% or so can be generally used. As the polyacrylamide gel to be used in the present invention, a gel with 15% to 20% or so is preferably used particularly for separating keratin proteins, etc., with higher resolution.

Operation of the SDS-PAGE method is not particularly limited, and a general method may be used. For example, with regard to a buffer solution for electrophoresis or power distributing conditions, these conditions are preferably set in consideration with resolution and sharpness of the electrophoretic pattern. In addition, for obtaining the electrophoretic pattern, an internal standard is preferably used in combination. As the internal standard, for example, bovine serum albumin, etc., is used for relative mobility calibration of the electrophoretic pattern.

The gel after the electrophoresis is stained the protein using a dyeing liquid. For dyeing, a dye staining or a silver staining, etc., is used. As the dye to be used, there may be mentioned, for example, Coomassie Brilliant Blue-R (CBB-R), etc. The gel thus stained shows an electrophoretic pattern (also referred to as "gel image".) constituted by a plural number of the bands having different mobilities due to the molecular weights.

(1-3) First discrimination process

The electrophoretic pattern (gel image) thus obtained is specific to the kinds of the original animals of the animal hair fibers. In this first embodiment, an electrophoretic pattern obtained by the electrophoretic process is compared to the electrophoretic patterns of several kinds of the single animal hair fibers in which the kinds of the original animals have been already known.

Thus, with regard to the several kinds of the single animal hair fibers in which the kinds of the animals have been already known, electrophoretic patterns are previously prepared by the same procedure as mentioned above. Also, when the electrophoretic patterns of the animal hair fibers sample is prepared, the electrophoretic patterns of several kinds of the single animal hair fibers in which the kinds of the original animals have been already known may be simultaneously prepared. By employing such a procedure, more accurate discrimination can be done.

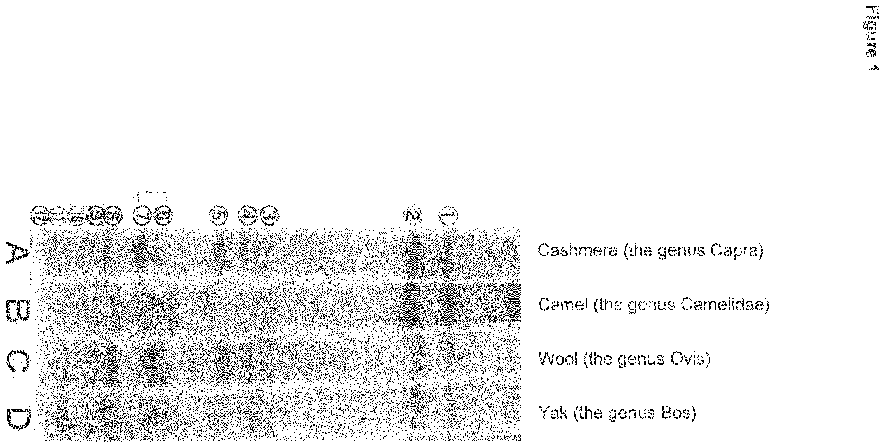

As an example, specifically prepared electrophoretic patterns of 4 kinds of the animal hair fibers in which the kinds of the animals have been already known are shown in Fig. 1. Fig. 1 shows electrophoretic patterns (gel images) of A: cashmere (the genus Capra), B: camel (the genus Camelidae), C: wool (the genus Ovis) and D: yak (the genus Bos). In Fig. 1, the dark portion of the stained intensity is a band containing a protein. In Fig. 1, the upper band is a smaller relative mobility and has a larger molecular weight. On the other hand, the lower band is a larger relative mobility and has a smaller molecular weight.

In Fig. 1, A: cashmere (the genus Capra) and the other animal hair fibers are compared to each other, even in the discrimination with visual observation, B: camel (the genus Camelidae) has markedly different band pattern as compared to that of A: cashmere (the genus Capra). In addition, D: yak (the genus Bos) has light intensities of the bands 3, 4, 5 and 7 and the band 8, and dark intensities of the band 9 and the band 12 as compared to those of A: cashmere (the genus Capra). Also, C: wool (the genus Ovis) has a narrow distance between the band 6 and the band 7, and has no band 12 as compared to those of A: cashmere (the genus Capra).

Here, a method for more objectively comparing the obtained electrophoretic patterns is explained. First, gel images of the obtained electrophoretic patterns are incorporated by a scanner, the position (corresponding to the molecular weight) of is quantified as a relative mobility Rf, and the intensity (corresponding to the protein amount) of each band is quantitatively determined. At this time, the relative mobility Rf of the bands between a plural number of samples is preferably corrected.

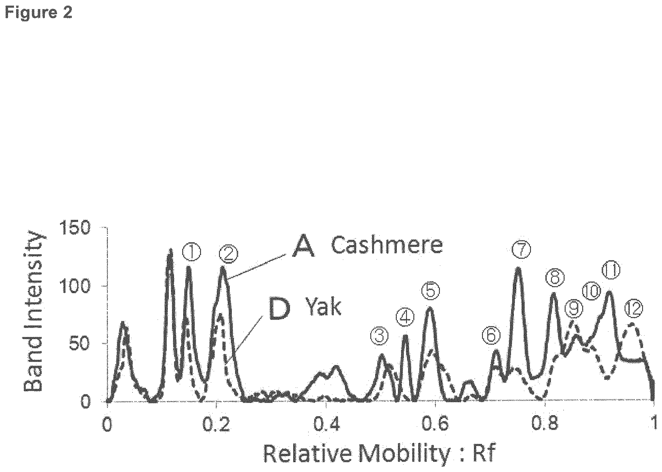

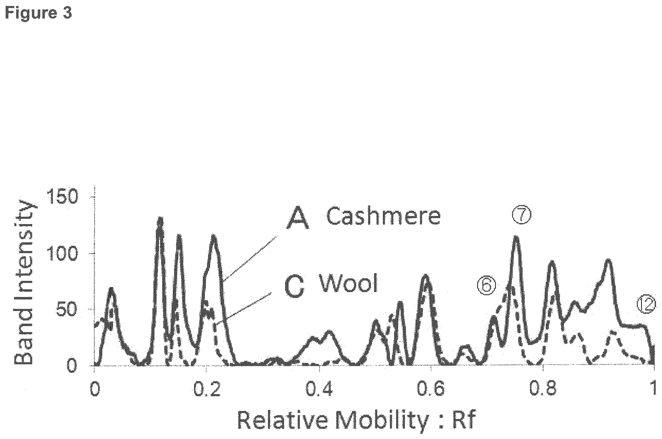

Each electrophoretic pattern (gel image) shown in Fig. 1 is incorporated by a scanner, the position and the intensity of each band are quantitatively determined, and an analyzed chart showing the relation between the relative mobility Rf and the band intensity (peak) is shown in Fig. 2 and Fig. 3. In Fig. 2 and Fig. 3, the horizontal axis is the relative mobility Rf (corresponding to the molecular weight), and the right side means a larger relative mobility Rf and a smaller molecular weight. The vertical axis is the band intensity (corresponding to the protein amount) of each band, and when the peak is high and the area is large, the protein amount is much.

Here, analyzed charts of yak (the genus Bos) and wool (the genus Ovis) which are mixed for camouflage purpose in many cases are compared with an analyzed chart of cashmere (the genus Capra). Fig. 2 is a drawing comparing the electrophoretic patterns (analyzed charts) of A: cashmere (the genus Capra) and D: yak (the genus Bos). It has clearly been shown that D: yak (the genus Bos) has light intensities of the bands 3, 4, 5 and 7 and the band 8, and has dark intensities of the band 9 and the band 12 as compared with those of A: cashmere (the genus Capra).

In addition, Fig. 3 is a drawing comparing the electrophoretic patterns (analyzed charts) of A: cashmere (the genus Capra) and C: wool (the genus Ovis). It has clearly been shown that C: wool (the genus Ovis) has a narrow distance between the band 6 and the band 7 as compared with those of A: cashmere (the genus Capra), and has no band 12.

Thus, in this first embodiment, the electrophoretic pattern of the animal hair fibers sample is compared with the electrophoretic patterns of the single animal hair fibers in which the kinds of the original animals have been known, and by confirming the position and the band intensity, or the relative mobility Rf and the peak area of each band, the kinds of the original animals of the animal hair fibers sample can be discriminated. In particular, yak (the genus Bos) or wool (the genus Ovis) which is frequently mixed by camouflage with high class animal hair fibers such as cashmere (the genus Capra) can be clearly distinguished.

An additional object of the animal hair fibers sample is not fibers which are originated from a single animal, but fibers in which two or more kinds of the animal hair fibers are mixed. That is, in the first discrimination process, it is employed in the case where electrophoretic patterns of a plural number of the single animal hair fibers in which the kinds of the animals which have been already known are not matched to the electrophoretic pattern of the animal hair fibers sample, and the kinds of the animals cannot clearly be discriminated.

In addition , the mixed animal hair fibers in which the kinds of the original animals of the animal hair fibers are different have been mixed with a series of mixing ratios are prepared, and a series of the electrophoretic patterns thereof is previously prepared. In addition, in the second discrimination process, the electrophoretic pattern of the obtained animal hair fibers sample is compared with a series of the electrophoretic patterns of the previously prepared mixed animal hair fibers.

According to this procedure, it can be discriminated that the animal hair fibers sample are originated from a plural number of the animals. In addition, it can be also discriminated that the animal hair fibers of which kind of animals are mixed in the animal hair fibers sample, and further, how much these animal hair fibers are mixed with a mixing ratio.

In the following, the respective processes are explained in detail.

(1-4) Second discrimination process

The obtained electrophoretic pattern (gel image) changes in proportion with the mixing ratio of the animal hair fibers. The electrophoretic pattern of the animal hair fibers sample obtained by the electrophoretic process is compared with a series of the electrophoretic patterns of the previously prepared mixed animal hair fibers. For this procedure, the mixed animal hair fibers in which animal hair fibers having different kinds of the animals serving as the origin are mixed with a series of mixing ratios are prepared, and to these samples, a series of electrophoretic patterns are previously prepared according to the same procedure as mentioned above.

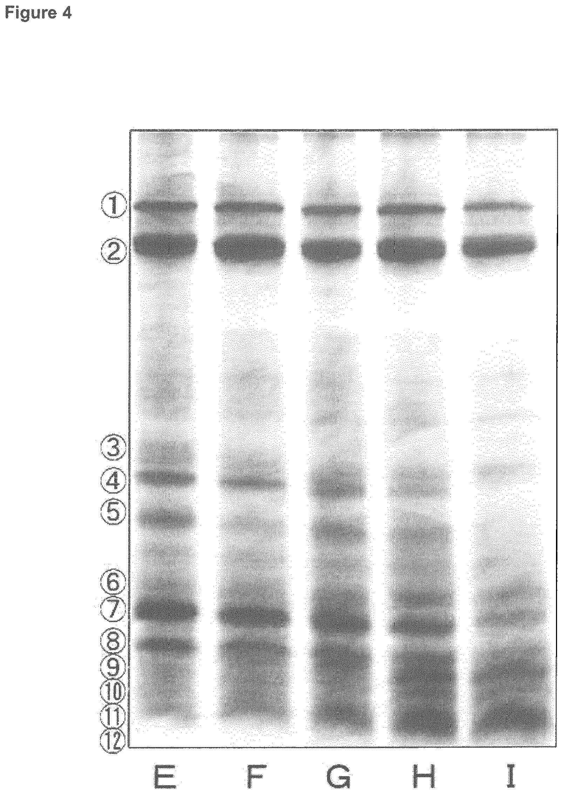

As an example, a series of electrophoretic patterns in which specifically prepared two kinds of the animal hair fibers have been mixed are shown in Fig. 4. Fig. 4 shows electrophoretic patterns (gel images) of a series of the mixed animal hair fibers in which, to the animal hair fibers originated from cashmere (the genus Capra) and yak (the genus Bos), a ratio of cashmere:yak is mixed and changed with a mixing ratio of E: 100% vs. 0%, F: 70% vs. 30%, G: 50% vs. 50%, H: 30% vs. 70%, I: 0% vs. 100%. In Fig. 4, the upper band is a smaller relative mobility and has a larger molecular weight. On the other hand, the lower band is a larger relative mobility and has a smaller molecular weight.

As explained above, an electrophoretic pattern (gel image) of 100% cashmere (the genus Capra) and an electrophoretic pattern (gel image) of 100% yak (the genus Bos) are clearly different, and it can be understood that electrophoretic patterns (gel images) in which they are mixed are gradually changed in the intensities of the respective bands in proportion with the mixing ratio (see Fig. 4).

Here, in order to compare the obtained electrophoretic pattern more objectively, in the same manner as mentioned above, the gel image of the obtained electrophoretic pattern is incorporated into a scanner, the position (corresponding to the molecular weight) of each band is quantified as a relative mobility Rf, and the band intensity (corresponding to the protein amount) of each band may be quantitatively determined.

Thus, by comparing the electrophoretic pattern of the animal hair fibers sample with the electrophoretic patterns of a series of the mixed animal hair fibers, and confirming the position and the band intensity, or the relative mobility Rf and the peak area of each band, it can be discriminated that the animal hair fibers sample is a material in which the animal hair fibers of cashmere (the genus Capra) and yak (the genus Bos) are mixed. In addition, the mixing ratio of the animal hair fibers of cashmere (the genus Capra) and yak (the genus Bos) can be also discriminated.

In the same manner, with regard to the mixed animal hair fibers other than cashmere (the genus Capra) and yak (the genus Bos), by previously preparing the series of electrophoretic patterns, respectively, the kinds of the original animals can be discriminated. In particular, it can be confirmed that yak (the genus Bos) or wool (the genus Ovis) which is frequently mixed by camouflage with high class animal hair fibers is mixed.

<<Second embodiment>>

The discrimination method of the animal hair fibers according to this second embodiment is to carry out discrimination of unknown animal hair fibers sample objectively and stably by measuring analytical data of the electrophoretic patterns explained in the above-mentioned first embodiment with regard to the many standard samples, and making it databased.

In this second embodiment, first, a group of proteins of many standard samples (the single animal hair fibers) the origin of the animal hair fibers of which is clear, and many standard sample (the mixed animal hair fibers) in which the origin of the animal hair fibers and the mixing ratio of which are clear are extracted in the same manner as in the above-mentioned first embodiment (extraction process), and then, the extracted groups of proteins are separated by an electrophoresis apparatus to obtain electrophoretic patterns (electrophoretic process).

With regard to the many electrophoretic patterns thus obtained, in the same manner as in the above-mentioned first embodiment, the gel images are incorporated into a scanner, the position (corresponding to the molecular weight) of each band is quantified as the relative mobility Rf, and the band intensity (corresponding to the protein amount) of each band is quantitatively determined as a peak area. Many numerical data thus obtained are databased with the histories of the standard samples, whereby objective discrimination can be carried out in the third discrimination process. The specific method is explained in Example mentioned later.

(2-1) Third discrimination process

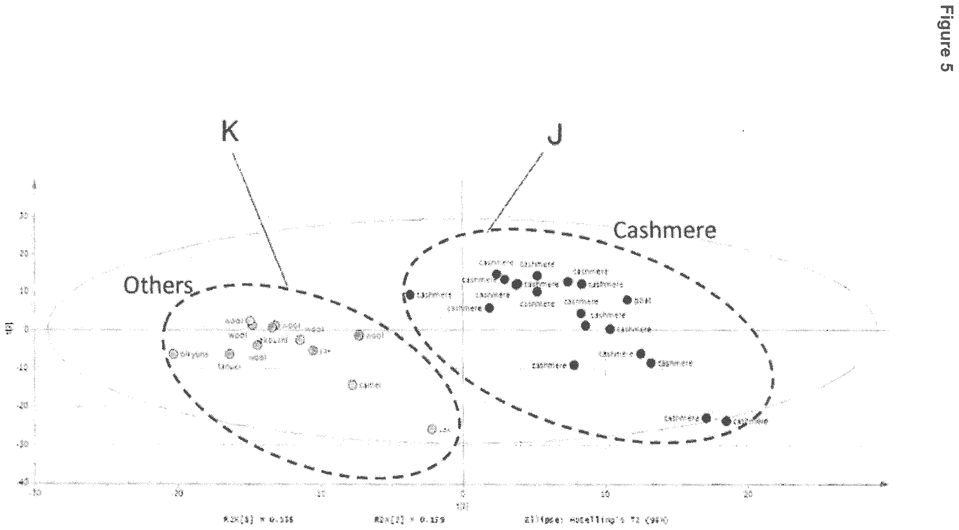

The thus constructed database is explained. Fig. 5 is a graph visualizing a part of the database prepared by a plural number of the standard samples with regard to cashmere (the genus Capra), camel (the genus Camelidae), wool (the genus Ovis), raw fiber (the single animal hair fibers) of yak (the genus Bos), and a mixture (mixed animal hair fibers) of cashmere (the genus Capra) and yak (the genus Bos). This Fig. 5 shows an example of the case which is classified into two groups of a group of J: cashmere (the genus Capra) and a group of K: other animal hair fibers (including the mixed animal hair fibers).

In this example, the numerical data obtained by analyzing the electrophoretic pattern of the animal hair fibers sample are collated with the database, and it can be discriminated whether the said animal hair fibers sample is cashmere (the genus Capra) or other animal hair fibers (including the mixed animal hair fibers).

In the same manner, by changing the classification of the database, it is possible to carry out the similar discrimination with regard to the animal hair fibers other than cashmere (the genus Capra). Further, by increasing an amount of the standard samples of the mixed animal hair fibers, it can be discriminated that whether any animal hair fibers are mixed to the animal hair fibers sample with any mixing ratio.

<<Third embodiment>>

An object of the discrimination method of the animal hair fibers according to this third embodiment is to further improve the discrimination precision in the respective discrimination methods of the above-mentioned first and second embodiment. More specifically, first, in the electrophoretic process, a polyacrylamide gel having higher resolution is used. Also, in the extraction process, a specific reducing agent which improves extraction efficiency of the protein is used. Further, "checking test" is used for improving the discrimination precision of the mixing ratio of the animal hair fibers sample by utilizing the clear electrophoretic pattern obtained by these. In the following, these are explained.

(3-1) Use of polyacrylamide gel having higher resolution

As explained in the above-mentioned first embodiment, in the electrophoretic process, the polyacrylamide gel electrophoresis method (SDS-PAGE method) using sodium dodecyl sulfate (SDS) as a support is preferably used. In this SDS-PAGE method, a polyacrylamide gel having higher resolution than the conventional ones is used, more accurate discrimination can be carried out.

Here, the gel having higher resolution means that it has good resolution at the region with a molecular weight of 25 kDa or less (hereinafter sometimes referred to as "protein region specific to animal species".), which is considered to be particularly important for discriminating animal species serving as the origin of the animal hair fibers in the electrophoresis method. More specifically, it means an electrophoretic gel having a resolution capable of separating 10 or more bands in the region with a molecular weight of 25 kDa or less to the electrophoretic pattern of at least the single animal hair fibers originated from cashmere.

A method for improving the resolution of the polyacrylamide gel is not particularly limited and, for example, the gel length (corresponding to the distance of the support) may be elongated to improve the resolution. In general, as the polyacrylamide gel to be used in the SDS-PAGE method, a gel having the gel length of 8 cm or so (hereinafter referred to as "8 cm-length gel".) is used. In this third embodiment, by changing the gel length from a gel with 8 cm to a gel with 12 cm (hereinafter referred to as "12 cm-length gel".), keratin protein, etc., can be separated with higher resolution.

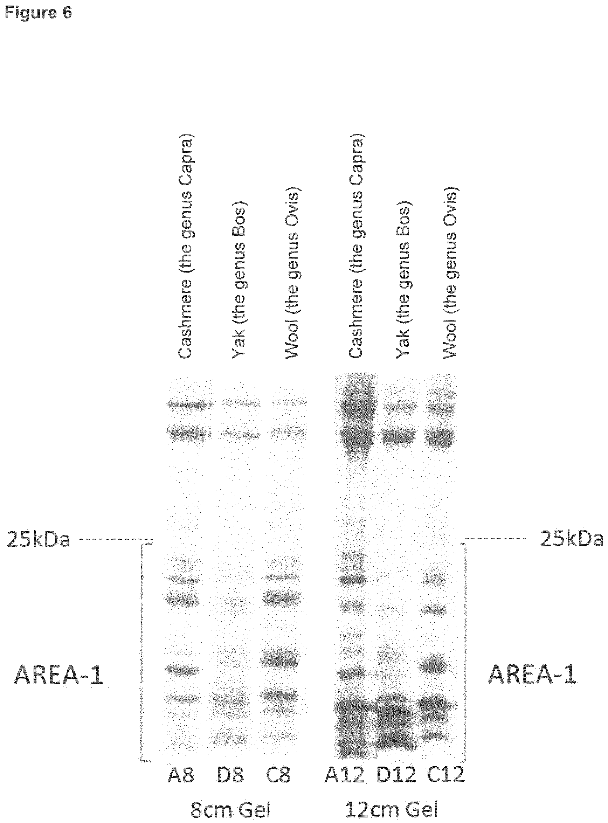

As an example, electrophoretic patterns (gel images) obtained by using a 8 cm-length gel and a 12 cm-length gel to three kinds of the animal hair fibers the kinds of the animals of which have been already known are shown in Fig. 6. Fig. 6 is a drawing comparing electrophoretic patterns (gel images) of A8: cashmere (the genus Capra), C8: wool (the genus Ovis) and D8: yak (the genus Bos) obtained by using a 8 cm-length gel generally used, and electrophoretic patterns (gel images) of A12: cashmere (the genus Capra), C12: wool (the genus Ovis) and D12: yak (the genus Bos) obtained by using a 12 cm-length gel having higher resolution.

Incidentally, in Fig. 6, the distances of the supports of each gel are to be visually accorded, the 12 cm-length gel is reduced in the lengthwise direction to correspond to the 8 cm-length gel. In Fig. 6, the dark portion of the stained intensity is a band containing a protein. In Fig. 6, the upper band has a smaller relative mobility and a larger molecular weight. On the other hand, the lower band has a larger relative mobility and a smaller molecular weight.

In the gel image of Fig. 6, the lower region having large relative mobility (the region having smaller molecular weight) is shown to be a protein region specific to animal species (AREA-1) effective for discrimination. This protein region specific to animal species (AREA-1) is a region having a molecular weight of 25 kDa or less. In Fig. 6, it can be understood that the resolution of the protein region specific to animal species (AREA-1) is improved in the 12 cm-length gel as compared with that of the 8 cm-length gel.

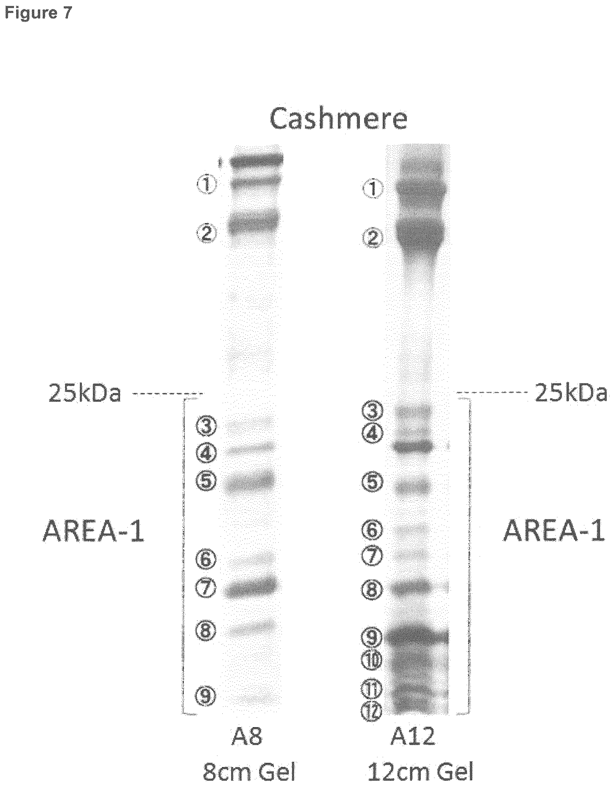

Fig. 7 is to compare the 8 cm-length gel and the 12 cm-length gel by extracting the electrophoretic patterns of cashmere (the genus Capra) among the electrophoretic patterns (gel images) in Fig. 6. In the gel images of Fig. 7, resolution of the protein region specific to animal species (AREA-1) is compared. In the electrophoretic patterns of cashmere (the genus Capra), in the case of the 8 cm-length gel, it is separated to 7 bands of the bands 3 to 9. To the contrary, in the case of the 12 cm-length gel, it is separated to 10 bands of the bands 3 to 12. According to this procedure, for example, by using the polyacrylamide gel having higher resolution such as the 12 cm-length gel, discrimination precision of the animal hair fibers sample can be improved.

Incidentally, even when the same 12 cm-length gel is used, a number of the bands in the region of 25 kDa or less is different depending on the animal species. However, in the cashmere (the genus Capra) which is particularly required to carry out discrimination, when a gel having higher resolution in which bands are separated to 10 or more is applied to the respective animal hair fibers, discrimination between the cashmere and the other animal hairs becomes easy.

(3-2) Use of reducing agent which improves extraction efficiency of protein

As explained in the above-mentioned first embodiment, in the extraction process of the protein, the disulfide bond (S-S bond) in the protein is reduced to split the same to make it as a thiol group, it is preferred to use a reducing agent with the extracting liquid. Also, as mentioned above, as these reducing agents, there may be mentioned, for example, 2-mercaptoethanol (2-ME), dithiothreitol (DTT), tris(2-carboxyethyl)phosphine (TCEP), etc.

In this third embodiment, among these reducing agents, 2-mercaptoethanol (2-ME) or dithiothreitol (DTT) is preferably used. Also, a concentration of the reducing agent to be used in the extracting liquid when 2-mercaptoethanol (2-ME) is used is preferably a concentration of higher than 6% in a concentration of % by volume, further preferably a concentration of higher than 10%. On the other hand, a concentration of the reducing agent to be used in the extracting liquid when dithiothreitol (DTT) is used is preferably a molar concentration of 8 to 12 mM (millimole/liter), further preferably substantially 10 mM (millimole/liter). By using these reducing agents with such a concentration, extraction efficiency of the protein in the extraction process is improved, and the electrophoretic pattern obtained in the subsequent electrophoretic process is clear whereby more accurate discrimination can be carried out.

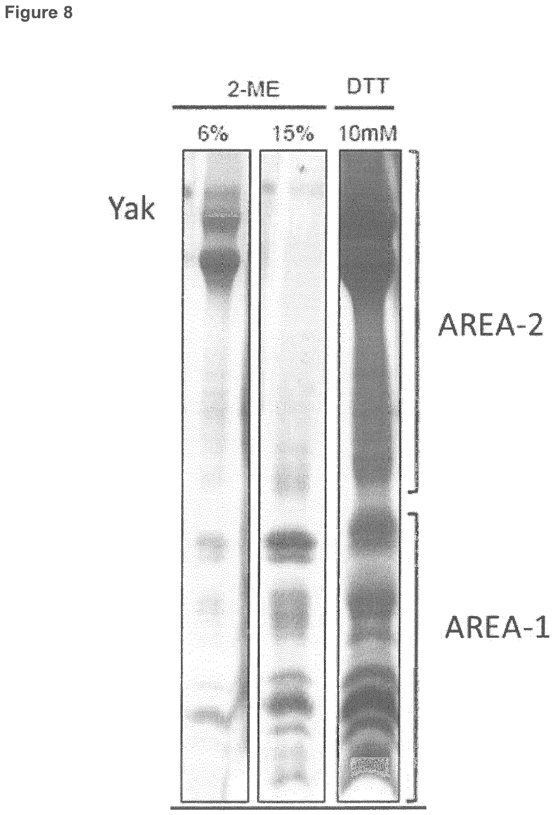

As an example, electrophoretic patterns (gel images) of the yak (the genus Bos) obtained by using 6% (conventional concentration) and 15% (concentration of this third embodiment) of 2-mercaptoethanol (2-ME) in a concentration of % by volume, and 10 mM (concentration of this third embodiment) of dithiothreitol (DTT) in a molar concentration, respectively, are shown in Fig. 8 (more specifically, it is mentioned later in Example).

(3-3) Use of "checking test"

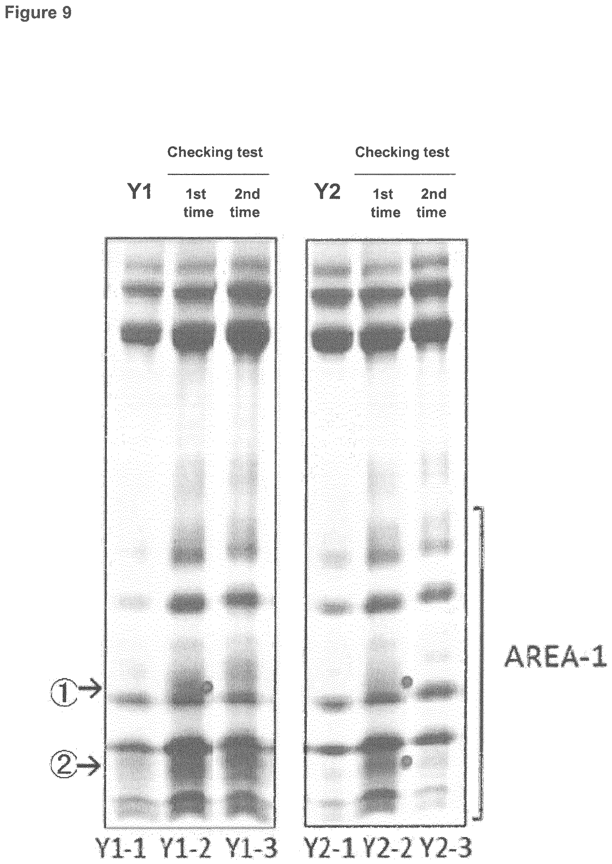

The "checking test" herein mentioned means a method for improving discrimination precision of the mixing ratio of the animal hair fibers sample obtained in the respective discrimination processes of the above-mentioned first embodiment or second embodiment. More specifically, first, electrophoretic patterns of the animal hair fibers sample (gel images) are obtained in the above-mentioned first embodiment or second embodiment. For example, the electrophoretic patterns (gel image Y1-1 and Y2-1) obtained in the two kinds of the animal hair fibers sample (Y1 and Y2) are shown in Fig. 9. From these gel images (Y1-1 and Y2-1), in the above-mentioned second discrimination process or the third discrimination process, a mixing ratio of the animal hair fibers sample is predicted (more specifically, it is mentioned later in Example). Next, by using a plural number of the single animal hair fibers in which the kinds of the original animals have been already known, comparative animal hair fibers in which the fibers are mixed with the same ratio with the predicted mixing ratio is prepared.

Next, with regard to the prepared comparative animal hair fibers, electrophoretic pattern (gel images Y1-2 and Y2-2 in Fig. 9) of the comparative animal hair fibers is obtained in the same manner as in the above-mentioned first embodiment or second embodiment. With regard to these gel images (Y1-2 and Y2-2), discrimination of the second discrimination process or the third discrimination process is again carried out to obtain a mixing ratio of the comparative animal hair fibers (more specifically, it is mentioned later in Example). According to these procedures, when the mixing ratio of the obtained comparative animal hair fibers is substantially matched with the mixing ratio (the same as the mixing ratio of the animal hair fibers sample firstly obtained) at the time of preparing the comparative animal hair fibers, accuracy of the mixing ratio of the animal hair fibers sample firstly obtained can be confirmed.

On the other hand, when the mixing ratio of the obtained comparative animal hair fibers is markedly different from the mixing ratio (the same as the mixing ratio of the animal hair fibers sample firstly obtained) at the time of preparing the comparative animal hair fibers, other comparative animal hair fibers in which the mixing ratio has been changed is again prepared. Next, electrophoretic patterns (gel images Y1-3 and Y2-3 in Fig. 9) of these other comparative animal hair fibers are obtained. To these gel images (Y1-3 and Y2-3), discrimination of the second discrimination process or the third discrimination process is again carried out to obtain the mixing ratios of the other comparative animal hair fibers (more specifically, it is mentioned later in Example). By repeating such operations, accuracy of the mixing ratio of the animal hair fibers sample can be improved. According to this procedure, discrimination of the more accurate mixing ratio can be carried out relatively simple and easy, and objectively.

<<Fourth embodiment>>

The discrimination method of the animal hair fibers according to this fourth embodiment has, in the discrimination method similar to the above-mentioned first embodiment, a fourth discrimination process which is to discriminate when the animal hair fibers sample is constituted by the specific two kinds of the animal hair fibers. In the fourth discrimination process, an object is to carry out discrimination of the mixing ratio of the animal hair fibers sample simply and easily, and with high precision by paying attention to the band specific to these animal hair fibers without making any database as in the above-mentioned second embodiment.

Incidentally, in the present invention, the specific two kinds of the animal hair fibers constituting the animal hair fibers sample is not particularly limited, it is necessary to exist a specific band which can differentiate these in the electrophoretic patterns of the respective animal hair fibers. In this fourth embodiment, it is explained by referring to the animal hair fibers sample in which two kinds of the animal hair fibers of cashmere (the genus Capra) and wool (the genus Ovis) have been mixed as an example.

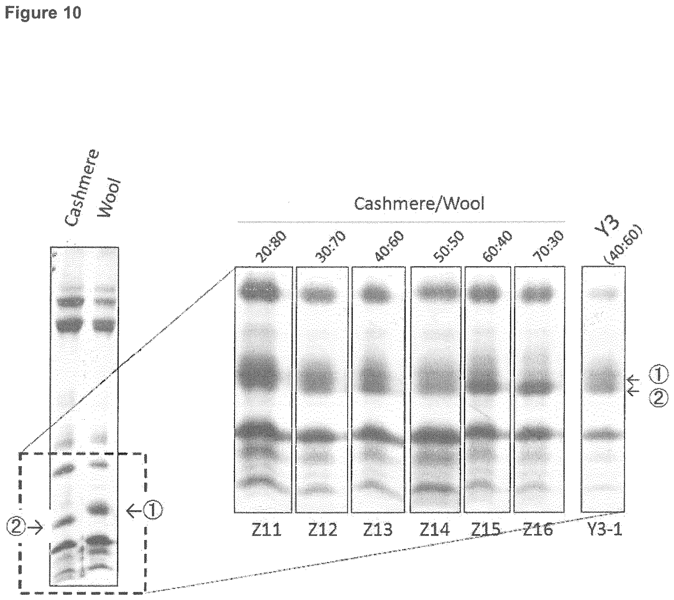

The electrophoretic pattern (A) of cashmere (the genus Capra) and the electrophoretic pattern (C) of wool (the genus Ovis) constituting the animal hair fibers sample are shown in Fig. 10. In Fig. 10, when the two electrophoretic patterns (A and C) are compared to each other, it can be understood that band 1 and band 2 are bands specific to cashmere (the genus Capra) and wool (the genus Ovis). When it has already been known that the animal hair fibers sample is constituted by cashmere (the genus Capra) and wool (the genus Ovis), discrimination of the mixing ratio of the animal hair fibers sample can be carried out simply and easily, and with high precision by paying attention to the band 1 and the band 2 without analyzing all the bands of the electrophoretic pattern.

(4-1) Fourth discrimination process

First, standard samples in which the mixing ratio of cashmere (the genus Capra) and wool (the genus Ovis) has been changed are prepared, and electrophoretic patterns (gel images Z1) thereof are obtained. In Fig. 10, gel images (Z11 to Z16) of the respective standard samples in which they are adjusted to cashmere:wool=20:80, 30:70, 40:60, 50:60, 60:40 and 70:30 are shown. In these gel images (Z11 to Z16), it can be understood that intensities of the band 1 and the band 2 are changed in response to the change in the mixing ratio of two kinds of the animal hair fibers.

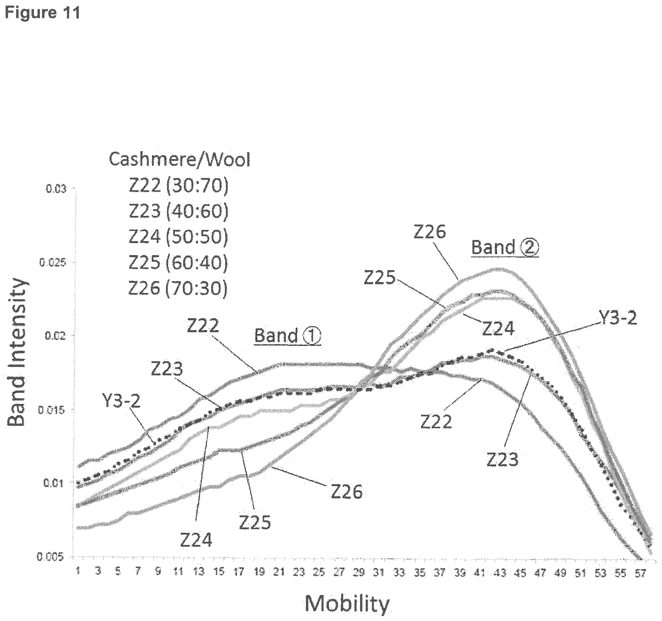

Next, an analyzed chart (Z2) showing the relation between the relative mobility Rf and the band intensity is prepared by analyzing these gel images (Z11 to Z16) (see Fig. 11). In Fig. 11, analyzed charts (Z22 to Z26) of the respective standard samples in which they are adjusted to cashmere:wool=30:70, 40:60, 50:60, 60:40 and 70:30 are shown. In these analyzed charts (Z22 to Z26), change in intensities of the band 1 and the band 2 can be clearly judged in response to the change in the mixing ratio of two kinds of the animal hair fibers. Thus, an analyzed chart showing the relation of the relative mobility Rf and the band intensity is obtained by analyzing the electrophoretic patterns to the animal hair fibers sample, and by comparing it with the analyzed charts (Z22 to Z26) of Fig. 11, discrimination of the mixing ratio of the animal hair fibers sample can be carried out simply and easily, and with high precision (more specifically, it is mentioned later in Example).

FURTHER ASPECTS OF THE DISCLOSURE

<<First further aspect of the disclosure>>

The discrimination method of the animal hair fibers according to this first further aspect of the disclosure comprises an extraction process, an electrophoretic process, a cutting process digestion process, a mass spectrometry process and a fifth discrimination process. In this first further aspect of the disclosure, in the same manner as in the above-mentioned first embodiment, first, a group of proteins is extracted from the animal hair fibers sample (extraction process), and the extracted group of proteins was separated by an electrophoresis apparatus to obtain an electrophoretic pattern (electrophoretic process).

Further, in this first further aspect of the disclosure, from the electrophoretic pattern of the obtained animal hair fibers sample, a predetermined band among a plural number of the bands forming the said electrophoretic pattern is cut (cutting process). Next, the protein in the cut band is digested by a predetermined digestive enzyme to divide into a series of a peptide group (digestion process). Next, the obtained peptide group is analyzed by a mass spectrometer to obtain a mass spectrometric pattern (peptide pattern) due to the difference in the molecular weight of the respective peptides (mass spectrometry process).

Incidentally, as mentioned above, the electrophoretic pattern of the group of proteins extracted from the animal hair is specific to the kinds of the animals. In addition, a length of the peptide formed at the time of cutting these proteins at the specific amino acid portion or an amino acid sequence is also specific to the kinds of the animals. Based on the above as a basis, by comparing the mass spectrometric pattern of the obtained animal hair fibers sample with the mass spectrometric pattern of the several kinds of the animal hair fibers in which the kinds of the original animals have been known, the kinds of the original animals of the animal hair fibers sample are discriminated (fifth discrimination process).

In the following, the respective processes in this first further aspect of the disclosure are explained in detail. However, with regard to the extraction process and the electrophoretic process in this first further aspect of the disclosure are the same as in the above-mentioned first embodiment, and the explanation thereof is omitted herein. Incidentally, in this first further aspect of the disclosure, processes of a cutting process and the following processes can be carried out by utilizing the obtained electrophoretic pattern (gel sample) in the above-mentioned first embodiment.

(5-1) Cutting process

A predetermined band (gel section) is cut from the electrophoretic pattern (gel sample) obtained in the electrophoretic process among a plural number of the bands forming the said electrophoretic pattern. The band to be cut is required to be a band existing at the common relative mobility Rf of the single animal hair fibers, if the single animal hair fibers to be an object for comparison has already been determined. Accordingly, with regard to the animal hair fibers sample the origin of which has not yet been known, it is preferred to be a band existing at the common relative mobility Rf to the main plural number of the single animal hair fibers.

In the present disclosure, in particular, there is a case where yak (the genus Bos) or wool (the genus Ovis) which is frequently mixed with the high class animal hair fibers such as cashmere (the genus Capra) with camouflage is clearly differentiated, so that it is more preferably a band existing at the relative mobility Rf common to these single animal hair fibers.

In addition, the band to be cut is preferably a band in which the molecular weight of the protein is not so large, for example, a band with the molecular weight of 30 kDa or less, preferably 20 kDa or less, further preferably around 10 kDa. When the molecular weight of the protein existing in the band is larger than 30 kDa, many peptides appear in the subsequent digestion process and resolution is lowered in the mass spectrometry process, whereby clear discrimination cannot be carried out.

From the fact as mentioned above, in the present disclosure, it is preferred to select and cut a band existing at the relative mobility Rf value common to the animal hair fibers of cashmere (the genus Capra), yak (the genus Bos), wool (the genus Ovis), and further adding camel (the genus Camelidae), and having a molecular weight of around 10 kDa with dark protein intensity.

(5-2) Digestion process

The treatment of the cut band (gel) is carried out by the general method. Next, a digestive enzyme is acted on the protein in the cut gel to cut to a peptide group. The method of acting the digestive enzyme on the protein in the gel is not particularly limited, and is generally preferably carried out a digestion in the gel. The digestive enzyme to be used in this digestion in the gel is not particularly limited, and may be used, for example, a digestive enzyme generally used for digestion of the protein such as trypsin, chymotrypsin, Lys-C, Glu-C, etc..

Incidentally, in the present disclosure, keratin protein, in particular, a protein having a small molecular weight is to be an object, so that it is preferred to use Lys-C which is a kind of an endoprotease cutting the protein from the C-side terminal of lysine which is a kind of an amino acid constituting the protein. By using Lys-C, an appearance amount of the peptide is suitable, and resolution is improved and clear discrimination can be carried out in the subsequent mass spectrometry process.

Thus, in this first further aspect of the disclosure, a protein having a relatively small molecular weight is cut in the cutting process, and further, a specific digestive enzyme of Lys-C is used, whereby precision of mass spectrometry is markedly improved.

(5-3) Mass spectrometry process

Next, to the obtained peptide group of the animal hair fibers sample, separation of the respective peptides and mass spectrometry are carried out. In this mass spectrometry process, various ionization means and mass detection method may be used in combination. As the ionization means, for example, MALDI (Matrix-assisted laser desorption/ionization), ESI (Electrospray ionization), NSI (Nano-spray ionization), etc., are utilized. Also, as the mass detection method, TOF (Time of flight) method, Ion trap method, Quadropole method, etc., are utilized.

In the present disclosure, a mass spectrometer (LC-MS) which is combined with liquid chromatography to be generally used for analysis of the peptide group or a Time of flight type mass spectrometer (TOF-MS) may be used. Also, as the other separating methods, the Cleveland method using the electrophoresis method, etc., may be used again.

Incidentally, in the present disclosure, nano liquid chromatography tandem mass spectrometer (nano-LC-MS/MS) in which NSI with high S/N ratio and liquid chromatography are combined, etc., may be preferably used.

Thus, a mass spectrometric pattern due to the difference in the molecular weight to the peptide group of the animal hair fibers sample can be obtained. The obtained mass spectrometric pattern is constituted by a plural number of peaks with different retention time by the molecular weight.

(5-4) Fifth discrimination process

The mass spectrometric pattern thus obtained is specific to the kinds of the original animals of the animal hair fibers. In this first further aspect of the disclosure, the mass spectrometric pattern obtained by the mass spectrometry process is compared with the mass spectrometric patterns of several kinds of the single animal hair fibers in which the kinds of the original animals have been known.

Thus, with regard to several kinds of the single animal hair fibers in which the kinds of the animals have been known, mass spectrometric patterns are previously prepared in the same procedures as mentioned above. Also, when the mass spectrometric pattern of the animal hair fibers sample is to be prepared, mass spectrometric patterns of several kinds of the single animal hair fibers in which the kinds of the original animals have been known may be prepared simultaneously. By taking the procedure as mentioned above, more accurate discrimination can be carried out.

As an example, mass spectrometric patterns of the specifically prepared four kinds of the animal hair fibers in which kinds of the animals have been known are shown in Fig. 6. Fig. 6 shows mass spectrometric patterns of L: cashmere (the genus Capra), M: camel (the genus Camelidae), N: wool (the genus Ovis) and O: yak (the genus Bos). In Fig. 6, as the peak at the left side, the retention time is short and the molecular weight of the peptide is small. On the other hand, as the peak at the right side, the retention time is long and the molecular weight of the peptide is large.

In Fig. 6, in the visual discrimination, mass spectrometric patterns of L: cashmere (the genus Capra), M: camel (the genus Camelidae), N: wool (the genus Ovis) and O: yak (the genus Bos) each has a characteristic peak, and difference due to the kinds of the animals is clear.

Here, the method of more objectively comparing the obtained mass spectrometric pattern is explained. First, with regard to the obtained mass spectrometric patterns, an area value (corresponding to the peptide amount) of each peak to the position (the retention time: corresponding to hydrophobicity and the molecular weight) of each peak is quantitatively determined.

Based on these numerical data, for example, by comparing the relation between an area value of the peak with the specific retention time and a total area value, more objective discrimination can be carried out. Or else, the relation between an area value of the peak with the specific retention time and an area value of the peak with the different retention time may be compared. At this time, the retention time between a plural number of samples is preferably corrected.

Thus, in this first further aspect of the disclosure, the mass spectrometric pattern of the animal hair fibers sample is compared with the mass spectrometric pattern of the single animal hair fibers in which the kinds of the original animals have been known, and by confirming the relation between the position and the height of each peak, or the retention time and the area value, the kinds of the original animals of the animal hair fibers sample can be discriminated. In particular, yak (the genus Bos) or wool (the genus Ovis) which is frequently mixed with the high class animal hair fibers such as cashmere (the genus Capra) by camouflage can be clearly distinguished.

<<Second further aspect of the disclosure >>

The discrimination method of the animal hair fibers according to this second further aspect of the disclosure has, similar to the above-mentioned first further aspect of the disclosure , an extraction process, an electrophoretic process, a cutting process, a digestion process, a mass spectrometry process and a fifth discrimination process, and further has a sixth discrimination process. In this second further aspect of the disclosure, first, a group of proteins constituting the said animal hair fibers is extracted from the animal hair fibers sample (extraction process). Next, the extracted group of proteins was separated by an electrophoresis apparatus too obtain electrophoretic pattern due to the difference in the molecular weights (electrophoretic process).

Further, from the obtained electrophoretic pattern of the animal hair fibers sample, a predetermined band is cut among a plural number of bands forming the said electrophoretic pattern (cutting process). Next, the protein in the cut band is digested by a predetermined digestive enzyme to divide into a series of a peptide group (digestion process). Next, the obtained peptide group is separated and analyzed by a mass spectrometer to obtain a mass spectrometric pattern due to the difference in the molecular weight of the respective peptides (mass spectrometry process).

Incidentally, in this second further aspect of the disclosure, an object of the animal hair fibers sample is not fibers which are originated from a single animal, but fibers in which two or more kinds of the animal hair fibers are mixed. That is, in the fifth discrimination process of the above-mentioned first further aspect of the disclosure, it is employed in the case where mass spectrometric patterns of a plural number of the animal hair fibers in which the kinds of the original animals have been known are not matched to the mass spectrometric pattern of the animal hair fibers sample, and the kinds of the animals cannot clearly be discriminated.

In this second further aspect of the disclosure, the mixed animal hair fibers in which the kinds of the original animals of the animal hair fibers are different have been mixed with a series of the mixing ratio are prepared, and a series of the mass spectrometric patterns thereof is previously prepared. In addition, in the sixth discrimination process, the mass spectrometric patterns of the obtained animal hair fibers sample is compared with a series of the mass spectrometric patterns of the previously prepared mixed animal hair fibers.

According to this procedure, it can be discriminated that the animal hair fibers sample are originated from a plural number of the animals. In addition, it can be discriminated whether animal hair fibers of what kind of an animal are mixed with the animal hair fibers sample, and whether these animal hair fibers are mixed with any mixing ratio.

With regard to the extraction process and the electrophoretic process in this second further aspect of the disclosure, these are the same as the above-mentioned first embodiment, and with regard to the cutting process, the digestion process and the mass spectrometry process, these are the same as the above-mentioned first further aspect of the disclosure so that the explanation thereof is omitted herein.

(6-1) Sixth discrimination process

The obtained mass spectrometric pattern is constituted by a plural number of peaks having different retention time due to the molecular weight similarly to the above-mentioned fifth discrimination process. The mass spectrometric pattern changes in proportion to the mixing ratio of the kinds of the original animals of the animal hair fibers. In the same manner as in the above-mentioned fifth discrimination process, with regard to the obtained mass spectrometric pattern, an area value (corresponding to the peptide amount) of each peak to the position (the retention time: corresponding to hydrophobicity and the molecular weight) of each peak is quantitatively determined.

Based on these numerical data, for example, by comparing the relation between an area value of the peak with the specific retention time and a total area value, objective discrimination can be carried out. Or else, the relation between an area value of the peak with the specific retention time and an area value of the peak with the different retention time may be compared.

Thus, in this second further aspect of the disclosure, the mass spectrometric pattern of the animal hair fibers sample is compared with the mass spectrometric patterns of a series of the mixed animal hair fibers, and by confirming the relation between the retention time and the area value of each peak, it can be discriminated that the animal hair fibers sample are originated from a plural number of the animals. In addition, it can be accurately discriminated whether animal hair fibers of what kind of an animal are mixed with the animal hair fibers sample, and whether these animal hair fibers are mixed with any mixing ratio.

<< Third further aspect of the disclosure >>

The discrimination method of the animal hair fibers according to this third further aspect of the disclosure is to carry out discrimination of unknown animal hair fibers sample objectively and stably, by measuring analytical data of the mass spectrometric patterns explained in the above-mentioned first further aspect of the disclosure with regard to the many standard samples, and making it databased.

In this third further aspect of the disclosure, first, with regard to many standard samples (the single animal hair fibers) the origin of the animal hair fibers of which is clear, and many standard sample (the mixed animal hair fibers) in which the origin of the animal hair fibers and the mixing ratio of which are clear, the peptide group is mass analyzed in the same manner as in the above-mentioned first further aspect of the disclosure to obtain mass spectrometric patterns (mass spectrometry process).

To the obtained many mass spectrometric patterns, in the same manner as in the above-mentioned first further aspect of the disclosure, an area value (corresponding to the peptide amount) of each peak to the position (the retention time: corresponding to hydrophobicity and the molecular weight) of each peak is quantitatively determined. Many numerical data thus obtained are databased with the histories of the standard samples, whereby objective discrimination can be carried out in the seventh discrimination process. The specific method is explained in Example mentioned later.

(7-1) Seventh discrimination process

The thus constructed database is explained. First, with regard to raw fibers (the single animal hair fibers) of cashmere (the genus Capra), camel (the genus Camelidae), wool (the genus Ovis) and yak (the genus Bos), and a mixture (the mixed animal hair fibers) of cashmere (the genus Capra) and yak (the genus Bos), database are constructed from the numerical data of a plural number of standard samples, respectively.

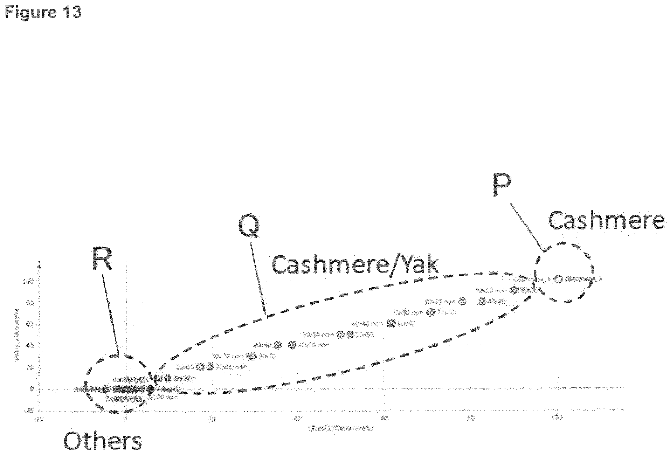

As an example, there are classified into three groups of a group of cashmere (the genus Capra), a group of the mixture of cashmere (the genus Capra) and yak (the genus Bos) and a group of other animal hair fibers. Here, to visualize the data, a graph taking the known mixing ratio of these groups at the vertical axis, and the mixing ratio predicted from the database at the horizontal axis is shown in Fig. 13.

This Fig. 13 is classified into three groups of a group of P: cashmere (the genus Capra), a group of the mixture of Q: cashmere (the genus Capra) and yak (the genus Bos) and a group of R: other animal hair fibers. Further, in Fig. 13, the mixing ratio of cashmere (the genus Capra) and yak (the genus Bos) is appeared accurately on a straight line.

In this example, by analyzing the mass spectrometric pattern of the animal hair fibers sample and collating the obtained numerical data with the database, it can be discriminated whether the said animal hair fibers sample is cashmere (the genus Capra), a mixture of cashmere (the genus Capra) and yak (the genus Bos), or other animal hair fibers. Further, when the animal hair fibers sample is a mixture of cashmere (the genus Capra) and yak (the genus Bos), the mixing ratio thereof can be discriminate with good accuracy.

In the same manner, by changing the classification of the database, the animal hair fibers other than cashmere (the genus Capra), and the mixed animal hair fibers other than cashmere (the genus Capra) and yak (the genus Bos) can be similarly discriminated.

In the following, the discrimination methods of the animal hair fibers according to the above-mentioned respective embodiments and aspects of the disclosure are specifically explained by referring to the respective Examples. First, among the respective Examples, in Example 1 to Example 3, 92 kinds of standard samples, and 3 kinds of the animal hair fibers sample (hereinafter also referred to as "sample to be tested".) shown below were prepared as the animal hair fibers.

First, as the standard samples of the single animal hair fibers the origins of which are clear, raw fibers (CCMI Fiber Box: Cashmere and Camel Hair Manufactures Institute) of cashmere (62 kinds), yak (10 kinds), wool (9 kinds) and camel (3 kinds) were prepared. Also, as the standard sample of the mixed animal hair fibers the origins and the mixing ratios of which are clear, samples in which raw fibers (CCMI) of cashmere and yak had been mixed to be a weight ratio of 9:1, 8:2, 6:4, 5:5, 4:6, 3:7, 2:8 and 9:1 were prepared.

Next, as the sample 1 to be tested, single animal hair fibers (TOYOBOSHI KOGYO CO., LTD.) in which cashmere has been dye-processed, and as the sample 2 to be tested, single animal hair fibers (DoubleTree Co., Ltd.) in which yak has been dye-processed were prepared. Also, as the sample 3 to be tested, mixed animal hair fibers in which raw fibers (CCMI) of cashmere and yak have been mixed with a weight ratio of 7:3 were prepared. Incidentally, the origin of the sample to be tested was tested in a manner not to known to the inspector.

Example 1

This Example 1 is to discriminate three kinds of the samples to be tested by the electrophoretic pattern based on the above-mentioned first embodiment.

(1) Extraction process

With regard to the dye-processed samples 1 and 2 to be tested (each single animal hair fibers), washing operation was carried out before extracting the protein. On the other hand, with regard to the sample 3 to be tested (the mixed animal hair fibers), protein extraction was carried out without effecting the washing operation. First, the washing operation was carried out by adding 250 $\mu$L of a washing solution (12 mM-deoxycholic acid , 12 mM-lauroyl sarcosinic acid, 100 mM-tris-hydrochloric acid buffer solution (pH 9.0)) to 10 mg of the sample. The sample was suspended in the washing solution so that it is dipped therein, and the sample was heated at 95°C for 20 minutes. Thereafter, the sample was centrifuged at 15,000×g for 10 minutes, and the supernatant was removed. The above-mentioned washing operation was carried out three times in total.

Extraction of the protein was carried out by adding 250 $\mu$L of an extracting liquid (2%-SDS, 6%-2-ME, 125 mM-tris-hydrochloric acid buffer solution (pH 6.8)) to 10 mg of the sample, and heating at 95°C for 20 minutes. Thereafter, the sample was centrifuged at 15,000×g for 10 minutes, and the supernatant was recovered as the protein extracted liquid. The extraction operation was repeated twice in total.

Before electrophoresis, acetone precipitation was carried out to concentrate the protein. The acetone precipitation was so carried out that, first, 3-fold amount of cold acetone was added to the amount of the sample and the mixture was stirred. The sample was allowed to stand at -80°C for 3 hours, and then, centrifuged at 15,000×g for 10 minutes at 4°C. After the supernatant was removed, the sample was dried by a centrifugal concentrator CC-105 (Tomy Seiko Co., Ltd.). The protein was dissolved in 60 $\mu$L of an extracting liquid (2%-SDS, 6%-2-ME, 125 mM-tris-hydrochloric acid buffer solution (pH 6.8)).

Thereafter, for alkylating the cysteine side chain, 6 $\mu$L of 500 mM iodoacetamide (IAA) was added to the sample, and the mixture was incubated under dark place at room temperature for 30 minutes. An amount of the extracted protein was quantitated by using 660 nm Protein Assay Kit (Thermo Fisher Scientific K.K.). Incidentally, as a standard sample (internal standard) for quantitative determination, bovine serum albumin (BSA, Wako Pure Chemical Industries, Ltd.) was used.

(2) Electrophoretic process

Next, with regard to each group of the proteins extracted from the samples 1 to 3 to be tested, electrophoresis was carried out as follows, respectively. For the electrophoresis, 20 ug of the protein was used. Before the electrophoresis, the sample was mixed with an equi-amount of a sample-preparing liquid for electrophoresis (4%-SDS, 12%-2-ME, 250 mM-tris-hydrochloric acid buffer solution (pH 6.8), 20%-glycerol, 0.02%-bromophenol blue), and mixed with 1 ng of BSA (internal standard), then, heated at 95°C for 5 minutes. As the electrophoresis tank, BE-211 (Bio Craft Co., Ltd.) was used, and the proteins were separated by using 20% SDS-polyacrylamide gel (TEFCO K.K.: 8 cm-length gel) .

The electrophoresis was carried out by 25 mA of the constant current per one sheet. As a molecular weight marker, 3 $\mu$L of BlueStar Prestained Protein Marker (NIPPON Genetics Co., Ltd.) was used for the electrophoresis. The gel after the electrophoresis was shaken with a 7.5%-acetic acid solution for 15 minutes, and then, shaped with a dyeing liquid (0.25%-CBB-R, 50%-methanol, 10%-acetic acid) for 20 minutes. For decoloring operation, 5%-methanol and 7%-acetic acid solution were used.

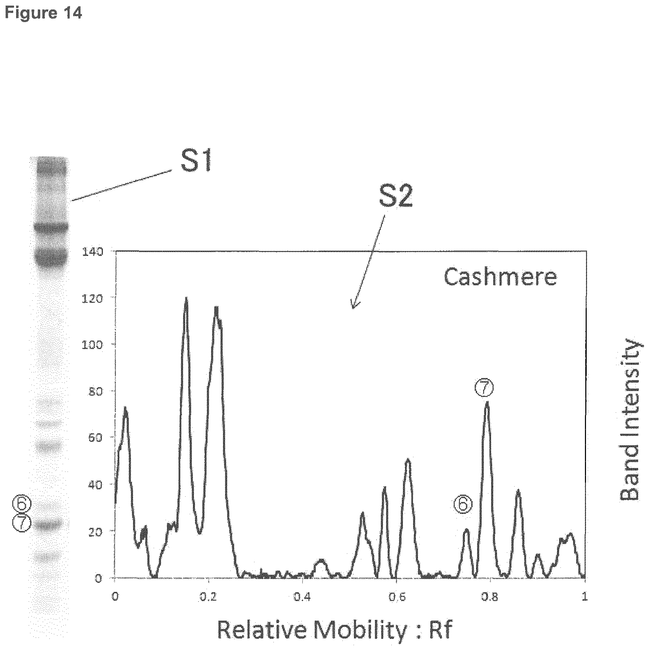

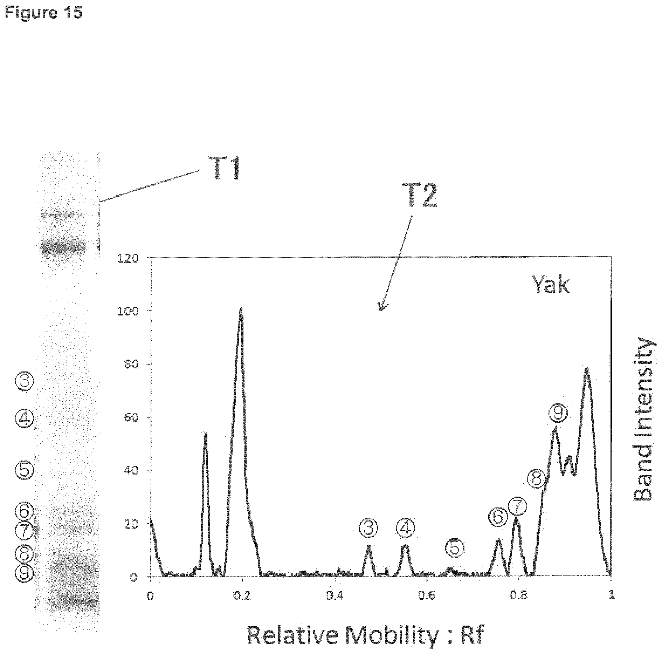

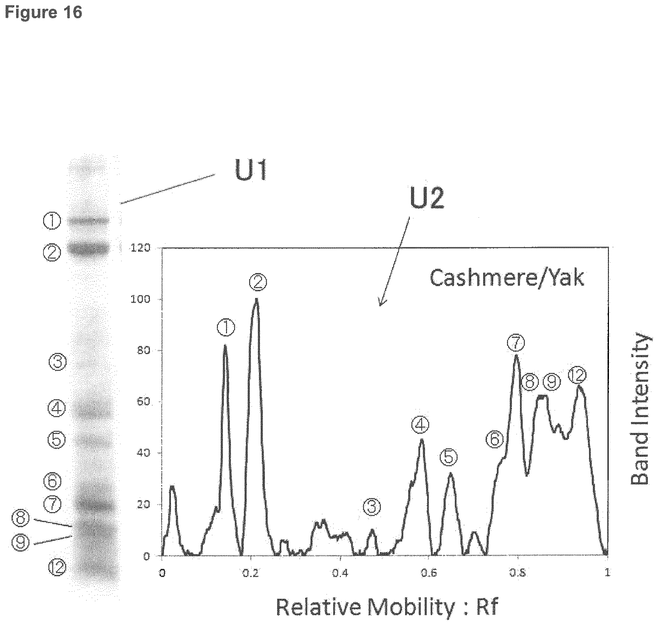

According to these procedures, electrophoretic patterns of the samples 1 to 3 to be tested were obtained. The obtained electrophoretic pattern of the sample 1 to be tested is shown in Fig. 14. In Fig. 14, a gel image is shown by S1, and an analyzed chart is shown by S2. Also, the obtained electrophoretic pattern of the sample 2 to be tested is shown in Fig. 15. In Fig. 15, a gel image is shown by T1, and an analyzed chart is shown by T2. Further, the obtained electrophoretic pattern of the sample 3 to be tested is shown in Fig. 16. In Fig. 16, a gel image is shown by U1, and an analyzed chart is shown by U2.

(3) Discrimination process

Next, the obtained electrophoretic pattern of the samples 1 to 3 to be tested were compared with the electrophoretic patterns of the standard samples. As the electrophoretic patterns of the standard samples, electrophoretic patterns of A: cashmere, B: camel, C: wool and D: yak shown in the above-mentioned Fig. 1 to Fig. 3 were used. In the electrophoretic pattern of the sample 1 to be tested, the band 7 is darker than that of the band 6, and the distance of the both bands is wider than that of the standard sample C: wool, so that the sample 1 to be tested was judged to be cashmere.

Also, in the electrophoretic pattern of the sample 2 to be tested, the intensities of the bands 3, 4, 5 and 6 were the same as that of the band 7, and the band 9 was darker than the band 8, so that the sample 2 to be tested was judged to be yak. On the other hand, in the electrophoretic pattern of the sample 3 to be tested, band 1, the intensities of the bands 3, 4, 5 and 6 to that of the band 2 were thinner than the standard sample A: cashmere, and the intensities of the band 9 and the band 12 to the band 8 were strong, so that the sample 3 to be tested was judged to be a mixture of cashmere and yak.

Example 2

This Example 2 is to discriminate three kinds of the samples to be tested by the database of the electrophoretic patterns based on the above-mentioned second embodiment.

First, construction of the database is explained. With regard to the above-mentioned standard sample 92 kinds, the gel images were each incorporated into a scanner. The gel images were scanned by a Basic Quantifier (Bio Image Systems Inc.), the density (Intensity) of each band and the relative mobility Rf of the band were quantified. For correcting an error of the relative mobility Rf between the respective samples, the numerical values incorporated by the Basic Quantifier were read by Excel 2010 (Microsoft).

Thereafter, the relative mobilities Rf of the bands at about 70 kDa (BSA: internal standard), 60 kDa, 50 kDa, 20 kDa, 15 kDa and 10 kDa were extracted by each sample, compared to the relative mobility Rf of the standard sample (cashmere-A3, CCMI) to prepare an approximate curve (quadratic). By using the coefficient obtained by the approximate curve, the relative mobility Rf of the each sample was corrected. With regard to the intensity of the band, the intensity in each relative mobility Rf was corrected by the intensity of the total bands.

The numerical data of 92 kinds of the standard samples were classified by main component analysis using SIMCA ver. 13.0 (Umetrics). As a result of the main component analysis, it was classified into two groups of a group of cashmere and a group of other animal hair fibers (non-cashmere) (see Fig. 5).

Next, when the numerical data (relative mobility Rf and Intensity of the band) obtained by the electrophoretic patterns of the samples 1 to 3 to be tested in the same manner were provided to the model constructed in the above-mentioned database, the sample 1 to be tested was classified into the group of cashmere. On the other hand, the sample 2 to be tested and the sample 3 to be tested were classified into the group of other animal hair fibers (non-cashmere).

Example 3

This Example 3 is to discriminate three kinds of the samples to be tested by the database of the mass spectrometric patterns of the peptide group based on the above-mentioned third further aspect of the disclosure. Incidentally, in this Example 3, each gel sample obtained in the electrophoretic process of the standard samples and the samples to be tested in the above-mentioned Example 1 was used.

(1) Digestion process

The gel obtained in the electrophoretic process of the above-mentioned Example 1 and Example 2 was discolored, and from the gel after discoloration, a band at around 10 kDa was cut out. To the gel was added 100 μL of a fixing solution (50%-methanol, 5%-acetic acid), and the mixture was stirred by Deep Well Maximizer (M/BR-022UP, Taitec Corporation) (10 minutes, 25°C, 1,500 rpm). After the supernatant was removed, 100 μL of acetonitrile was added to the gel, and the mixture was stirred.

After the upper layer was removed, 100 μL of 10 mM-dithiothreitol (DTT) was added thereto, and the mixture was stirred by Deep Well Maximizer (10 minutes, 56°C, 1,500 rpm). After the supernatant was removed, 100 μL of IAA was added to the residue, and the resulting mixture was stirred by Deep Well Maximizer (10 minutes, 25°C, 1,500 rpm).

After the supernatant was removed, 100 μL of acetonitrile was added to the mixture, and the resulting mixture was stirred by Deep Well Maximizer (10 minutes, 25°C, 1,500 rpm). After the supernatant was removed, 100 μL of acetonitrile was again added to the residue, and the resulting mixture was stirred by Deep Well Maximizer (10 minutes, 25°C, 1,500 rpm). After the supernatant was removed, the gel was dried by a centrifugal concentrator CC-105 for 5 minutes.

To the gel was added 20 μL of a Lys-C solution (0.01 ug/μL, Wako Pure Chemical Industries, Ltd.), and the resulting mixture was allowed to stand at 4°C for 30 minutes. Thereafter, 70 μL of Lys-C solution was added thereto, and the resulting mixture was allowed to stand at room temperature for 65 minutes. To the mixture was added 20 μL of 50 mM-ammonium hydrogencarbonate solution, and allowed to stand at 37°C overnight. The supernatant was recovered after the digestion processing, 100 μL of 30%-acetonitrile and 3%-trifluoroacetic acid (TFA) were added to the gel. The gel was stirred by Deep Well Maximizer (10 minutes, 25°C , 1,500 rpm), and the supernatant was recovered. This operation was carried out again. To the gel was added 100 μL of acetonitrile, the resulting mixture was stirred by Deep Well Maximizer (10 minutes, 25°C, 1,500 rpm), and the supernatant was recovered. This operation was carried out again.

All the supernatants was recovered in the same vessel. The solvent was removed by a centrifugal concentrator CC-105, and the peptide was dissolved in 50 μL of 1% TFA. The peptide was desalted and concentrated by a mini-column packed with SDB-XC (3M Company). The sample solvent was removed by a centrifugal concentrator CC-105, and the peptide was dissolved in 10 μL of 5%-acetonitrile and 0.1%-TFA containing 6 kinds of synthetic peptides (20 fmol/μL/peptide).

(2) Mass spectrometry process

According to these procedures, the obtained the respective peptide groups were each analyzed by using the nano liquid chromatography tandem mass spectrometry (nano LC-MS/MS). LTQ-Orbitrap (Thermo Fisher Scientific K.K.) in which Untimate 3000 (Thermo Fisher Scientific K.K.) as a nano LC and HTC-PAL (CTC Analyzer) as an autosampler had been combined was used for the measurement. An analytical column packed with Reprosil C18 materials (3 μm, Dr. Maisch GmbH) was used.

5 μL of the sample was used for the analysis. A flow rate of the mobile phase was made 500 nL/min. The mobile phases A and B used were 0.5%-acetic acid, and 0.5%-acetic acid and 80%-acetonitrile, respectively. By using Mass Navigator v1.2 (Mitsui Knowledge Industry Co., Ltd.), information of the peptide and the fragmentation ion was extracted by Mascot Generic Format (MGF) from the mass spectrometry data.

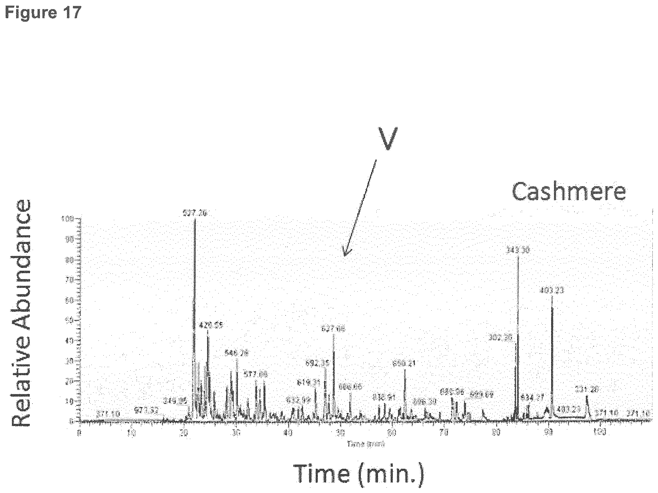

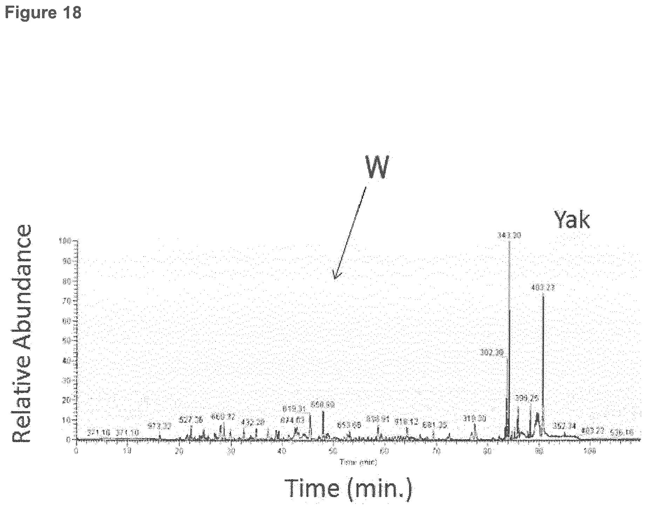

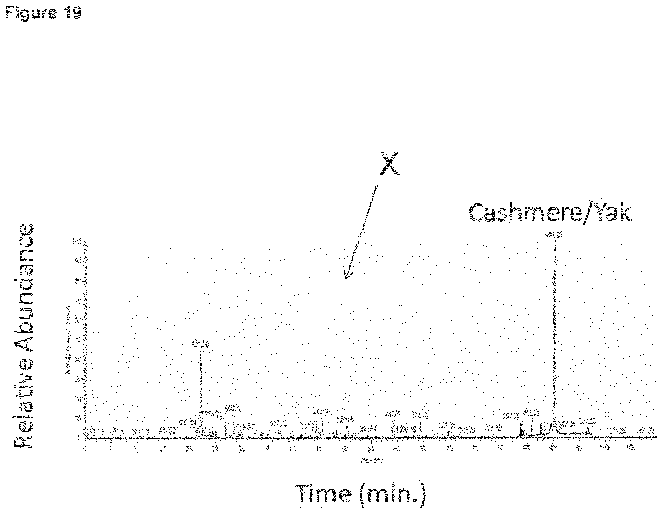

According to these procedures, mass spectrometric patterns of 92 kinds of the above-mentioned standard samples, and the samples 1 to 3 to be tested were obtained. The mass spectrometric pattern of the obtained sample 1 to be tested is shown in Fig. 17. The mass spectrometric pattern of the obtained sample 2 to be tested is shown in Fig. 18. The mass spectrometric pattern of the obtained sample 3 to be tested is shown in Fig. 19.

(3) Discrimination process

First, construction of the database is explained. With regard to the above-mentioned 92 kinds of the standard samples, Mascot v2.2 (Matrix Science Co.) was used for identifying the respective peptides, and an amount of the identified peptide (peak area value) was calculated by Mass Navigator v1.2. Fluctuation of the retention time of the peptide between the samples was corrected by using the retention time of the synthetic peptides. More specifically, the retention times of the synthetic peptides detected in each sample were compared to the retention time detected in the standard sample (cashmere-A3, CCMI) to prepare an approximate curve (quadratic). By using the coefficient obtained by the approximate curve, the retention time in each sample was corrected.

The peak area values of the respective peptides were corrected by the total peak area value. The numerical data of 92 kinds of standard samples were divided into groups by the main component analysis using SIMCA ver.13.0. As a result of the main component analysis, it was classified into three groups of a group of cashmere, a group of non-cashmere and a group of a mixture of cashmere and the other animal hair fibers (in this Example 3, a group of a mixture of cashmere and yak).

Next, when the numerical data (the retention time and the peak area value) obtained from the mass spectrometric patterns of the samples 1 to 3 to be tested in the same manner were provided to the model constructed in the above-mentioned database, the sample 1 to be tested was classified into the group of cashmere. Also, the sample 2 to be tested was classified into the group of non-cashmere. On the other hand, the sample 3 to be tested was classified into the group of a mixture of cashmere and yak.

Further, when the numerical data of the sample 3 to be tested were provided to the model (see Fig. 13) in which the already known mixing ratio was taken at the vertical axis and the mixing ratio predicted by the model was taken at the horizontal axis, the sample 3 to be tested was plotted to the position of a weight ratio of 7:3.

Thus, in the above-mentioned Example 1 to Example 3, the kinds of the original animals could be accurately discriminated with regard to the dye-processed samples 1 and 2 to be tested. Also, the kind and the mixing ratio of the mixed animal hair fibers could be accurately discriminated with regard to the sample 3 to be tested in which two kinds of the animal hair fibers have been mixed. These operations are relatively easy, and are an instrumental analysis, so that objective discrimination could be carried out without relying on the experience of an inspector or know-how.

From the above matter, in the present invention, the discrimination operation is relatively simple and easy, and has objectivity, discrimination can be carried out without relying on experience and know-how of an inspector, and even when a pre-treatment or dyeing processing has been done to the animal hair fibers, the discrimination method of the animal hair fibers which can obtain accurate discrimination results can be provided.

Next, Example 4 to Example 6 in which discrimination precision is improved based on the above-mentioned third embodiment, and Example 7 in which discrimination of the mixing ratio of the animal hair fibers sample (hereinafter also referred to as "the sample to be tested".) can be carried out simply and easily based on the fourth embodiment are explained.

Example 4

This Example 4 is to use a polyacrylamide gel having higher resolution based on the above-mentioned third embodiment (see Fig. 6 and Fig. 7). In this Example 4, by using electrophoretic patterns of 92 kinds of the samples the mixing ratios of which have been known obtained by using a 8 cm-length gel, and electrophoretic patterns of 79 kinds of the samples the mixing ratios of which have been known obtained by using a 12 cm-length gel, the database were constructed, respectively. By using the obtained respective databases, mixing ratios of five kinds of the samples to be tested the mixing ratios of which have been known were predicted.
Next, average prediction accuracy of the obtained respective databases was calculated. For the calculation of the average prediction accuracy, the following formula (1) was used.

$$\text{Average prediction accuracy} = \Sigma \left| \text{actual mixing ratio} - \text{predicted mixing ratio} \right| / \text{number of data} \cdots (1)$$

As a result, an average prediction accuracy ($\pm$standard deviation) when a 8 cm-length gel generally used had been used was 14.4$\pm$10.1%. To the contrary, an average prediction accuracy ($\pm$standard deviation) when a 12 cm-length gel had been used was 6.8$\pm$7.8%, whereby the average prediction accuracy improved about 2-fold as compared with the conventional ones by using the 12 cm-length gel having higher resolution.

Example 5

This Example 5 is to use a reducing agent which improves extraction efficiency of the protein based on the above-mentioned third embodiment. In this Example 5, as shown in Fig. 8, electrophoretic patterns (gel images) of yak (the genus Bos) were obtained by using 6% 2-mercaptoethanol (2-ME) (hereinafter referred to as "6%-2ME".) and 15% 2-mercaptoethanol (2-ME) (hereinafter referred to as "15%-2ME".) in a concentration of % by volume, and 10 mM dithiothreitol (DTT) (hereinafter referred to as "10 mM-DTT".) in a molar concentration, respectively (see Fig. 8).
In the gel images of Fig. 8, in contrast to the gel image obtained by 6%-2ME, in the gel image obtained by 15%-2ME, resolution at the lower region (protein region specific to animal species (AREA-1)) having a large relative mobility is improved, which shows that this region had been intensively extracted.
On the other hand, in the gel image obtained by 10 mM-DTT, extraction efficiency is improved as a whole not only at the protein region specific to animal species (AREA-1), but also at the upper region with small relative mobility (common protein region (AREA-2)).
Next, the mixing ratio was predicted from these electrophoretic patterns. For prediction of the mixing ratio, 6%-2ME was used and the database prepared by the 12 cm-length gel was used. The values of the mixing ratios predicted from the respective electrophoretic patterns are shown in Table 1.

[Table 1]

| Reducing agent | Predicted mixing ratio (%) | | |
|---|---|---|---|
| | Cashmere | Yak | Wool |
| 6%-2ME | 2.8 | 72.4 | 24.8 |
| 15%-2ME | -67.3 $\to$ 0 | 164.5 $\to$ 100 | 2.8 |
| 10 mM-DTT | -13.6 $\to$ 0 | 146.3 $\to$ 100 | -32.6 $\to$ 0 |
| Actual mixing ratio | 0 | 100 | 0 |

In this Example 5, the actual mixing ratio of the sample to be tested was 100% of yak. To the sample to be tested, the predicted mixing ratio obtained by 6%-2ME was 24.8% of wool. To the contrary, the predicted mixing ratio obtained by 15%-2ME was markedly improved as 2.8% of wool. In addition, the predicted mixing ratio of cashmere was below 0%, and the predicted mixing ratio of yak was above 100%. When the predicted mixing ratio was below 0%, it can be judged that the actual mixing ratio is 0% with few exception. Similarly, when the predicted mixing ratio was above 100%, it can be judged that the actual mixing ratio is 100% with few exception.

On the other hand, in the predicted mixing ratio obtained by 10 mM-DTT, the predicted mixing ratio of yak was above 100%, and the predicted mixing ratios of cashmere and yak were each below 0%. From these results, from the predicted mixing ratio obtained by 10 mM-DTT, it can be judged that it is 100% of yak.

From the results mentioned above, by using 15%-2ME or 10 mM-DTT as a reducing agent in the extracting liquid, discrimination precision of the sample to be tested can be improved.

Example 6

This Example 6 is to use "checking test" based on the above-mentioned third embodiment. In this Example 6, electrophoretic patterns of two kinds of the samples (Y1 and Y2) to be tested the mixing ratios of which have been known were obtained. In Fig. 9, the electrophoretic pattern of the sample (Y1) to be tested is shown by the gel image (Y1-1). Also, the electrophoretic pattern of the sample (Y2) to be tested is shown by the gel image (Y2-1).

In this Example 6, from these gel images (Y1-1 and Y2-1), the mixing ratios of the samples (Y1 and Y2) to be tested were predicted in the same manner as in the second discrimination process of the above-mentioned first embodiment. Next, by using a plural number of the single animal hair fibers in which the kinds of the original animals have been known, a first time comparative animal hair fibers (hereinafter referred to as "first comparative sample".) which has been mixed with the same ratio as the predicted mixing ratio was prepared.

Next, with regard to the prepared first comparative sample, electrophoretic patterns (Y1-2 and Y2-2) of the first comparative sample is obtained in the same manner as in the above-mentioned first embodiment (see Fig. 9). In this Example 6, the actual mixing ratio of the samples (Y1 and Y2) to be tested have been known. However, in the actual discrimination, the mixing ratio of the sample to be tested is unknown. Thus, in the actual discrimination, electrophoretic patterns (Y1-2 and Y2-2) obtained from the first comparative sample are compared with the electrophoretic patterns (Y1-1 and Y2-1) of the samples to be tested. If these electrophoretic patterns are substantially accorded, the predicted mixing ratio firstly obtained from the sample to be tested can be judged to be accurate.

On the other hand, if the electrophoretic patterns (Y1-2 and Y2-2) obtained from the first comparative sample are markedly different from the electrophoretic patterns (Y1-1 and Y2-1) of the samples to be tested, by using a plural number of the single animal hair fibers in which the kinds of the original animals have been known, a modified second time comparative animal hair fibers (hereinafter referred to as "the second comparative sample".) is again prepared by referring to the mixing ratio of the first comparative sample.

Next, with regard to the prepared second comparative sample, electrophoretic patterns (Y1-3 and Y2-3) of the second comparative sample were obtained in the same manner as in the above-mentioned first embodiment (see Fig. 9). The electrophoretic patterns (Y1-3 and Y2-3) obtained from the second comparative sample are compared with the electrophoretic patterns (Y1-1 and Y2-1) of the sample to be tested. Until these electrophoretic patterns are substantially matched, the same operation is repeated.

In Table 2, the values of the actual mixing ratio of the samples (Y1 and Y2) to be tested in this Example 6, the mixing ratio of each first comparative sample, and the mixing ratio of each second comparative sample are shown.

[Table 2]

| Sample to be tested | Mixing ratio % (cashmere:yak:wool) | | |
|---|---|---|---|
| | Actual mixing ratio | First comparative sample | Second comparative sample |
| Y1 | 60:25:15 | 55:10:35 | 60:30:10 |
| Y2 | 100:0:0 | 70:5:25 | 100:0:0 |

In Table 2, the actual mixing ratio (%) of the sample Y1 to be tested is cashmere:yak:wool=60:25:15. However, in the electrophoretic pattern obtained from the first comparative sample (cashmere:yak:wool=55:10:35), the band specific to wool (the band 1 in Fig. 9) is darker than that of the sample to be tested, so that the precision has not yet been good. To the contrary, the electrophoretic pattern of the second comparative sample prepared by referring to the mixing ratio (%) of the first comparative sample is similar to that of the sample to be tested (see Fig. 9). Thus, it can be understood that the mixing ratio (%) of the sample to be tested is closer to that of the second comparative sample (cashmere:yak:wool=60:30:10) than that of the first comparative sample, whereby discrimination precision is improved.

On the other hand, the actual mixing ratio (%) of the sample Y2 to be tested is cashmere:yak:wool=100:0:0. However, in the electrophoretic pattern obtained from the first comparative sample (cashmere:yak:wool=70:5:25), the band specific to wool (the band 1 in Fig. 9) and the band (the band 2 in Fig. 9) which exists in yak with a large amount are darker than those of the sample to be tested, whereby precision is not yet good. To the contrary, the electrophoretic pattern of the second comparative sample prepared by referring to the mixing ratio (%) of the first comparative sample is similar to that of the sample to be tested, and accorded to the actual mixing ratio (cashmere:yak:wool=100:0:0).

Thus, a new comparative sample is prepared based on the mixing ratio and its electrophoretic pattern used at the time of preparing the comparative sample, and the same operation is repeated until these electrophoretic patterns and the electrophoretic pattern of the sample to be tested are substantially accorded. By using the "checking test", accuracy of the mixing ratio of the sample to be tested can be improved. According to this procedure, more accurate discrimination of the mixing ratio can be carried out relatively simple and easy, and objectively.

Example 7

This Example 7 is to carry out discrimination of the mixing ratio of the sample to be tested simple and easily, and with high precision based on the above-mentioned fourth embodiment, when the sample to be tested is constituted by two kinds of the animal hair fibers of cashmere (the genus Capra) and wool (the genus Ovis) without making any database. In this Example 7, the electrophoretic pattern (A) of 100% cashmere and the electrophoretic pattern (C) of 100% wool constituting the sample (Y3) to be tested are shown in Fig. 10. In Fig. 10, when the two electrophoretic patterns (A and C) are compared to each other, it can be understood that the band 1 and the band 2 are bands specific to cashmere (the genus Capra) and wool (the genus Ovis).

Thus, standard samples in which the mixing ratio of cashmere (the genus Capra) and wool (the genus Ovis) have been changed were prepared, and their electrophoretic patterns (gel image Z1 of Fig. 10) were obtained. In Fig. 10, gel images (Z11 to Z16) of the respective standard samples in which they are adjusted to cashmere:wool=20:80, 30:70, 40:60, 50:60, 60:40 and 70:30 are shown. In these gel images (Z11 to Z16), it can be understood that intensities of the band 1 and the band 2 are changed in response to the change in the mixing ratio of two kinds of the animal hair fibers.

Next, these gel images (Z1) are analyzed to prepare an analyzed chart (Z2) showing the relation between the relative mobility Rf and the intensity (see Fig. 11). In Fig. 11, analyzed charts (Z22 to Z26) of the respective standard samples in which they are adjusted to cashmere:wool=30:70, 40:60, 50:60, 60:40 and 70:30 are shown. In these analyzed charts (Z22 to Z26), change in intensities of the band 1 and the band 2 can be clearly judged in response to the change in the mixing ratio of cashmere and wool.

In this Example 7, electrophoretic pattern is obtained with regard to the sample (Y3) to be tested the mixing ratio of which has already been known (gel image Y3-1 of Fig. 10). Next, this gel image (Y3-1) is analyzed to obtain an analyzed chart (Y3-2) showing the relation between the relative mobility Rf and the band intensity (see Fig. 11). Next, the obtained analyzed chart (Y3-2) of the sample (Y3) to be tested is compared with the analyzed charts (Z22 to Z26) of the respective standard samples previously obtained.

In Fig. 11, it can be confirmed that intensities (vertical axis of Fig. 11) of the band 1 and the band 2 have been changed by the mixing ratio of cashmere and wool. It can be understood that as the mixing ratio of cashmere increases, the intensity of the band 1 is thin, and accompanied thereby, the intensity of the band 2 is dark. Thus, when the analyzed chart (Y3-2) of the sample (Y3) to be tested and the analyzed charts (Z22 to Z26) of the respective standard samples are compared, it can be confirmed that the analyzed chart (Y3-2) of the sample (Y3) to be tested overlaps with the analyzed chart (Z23) of the standard sample in which cashmere:wool=40:60.

Accordingly, in this Example 7, for analyzing the electrophoretic pattern, discrimination of the mixing ratio of the sample to be tested can be carried out simply and easily, and with high precision by attracting attention to the band 1 and the band 2 without subjecting to correction of the relative mobility Rf or analysis of the whole bands by the main component analysis.

From the above matter, in the present invention, the discrimination operation is relatively simple and easy, and has objectivity, discrimination can be carried out without relying on experience and know-how of an inspector, and even when pre-treatment or dyeing processing has been carried out to the animal hair fibers, the discrimination method of the animal hair fibers which can obtain accurate discrimination results can be provided.

Incidentally, for carrying out the present invention, not only the above-mentioned respective Examples, but the following various modified examples may be mentioned.

1. In the above-mentioned Example 2, correction of the intensity of the band is carried out by the ratio to the intensity of the total bands, but the invention is not limited thereby, and may be corrected by the ratio to the intensity of the specific band (for example, BSA: internal standard).

2. In the above-mentioned Example 3, correction of the peak area value is carried out by the ratio to the total peak area value, but the disclosure is not limited thereby, and may be corrected by the ratio to the peak area value of the specific peptide.

3. In the above-mentioned Example 3, analysis of the peptide group is carried out by LC-MS/MS, but the disclosure is not limited thereby, and a UV visible spectroscopic detector may be employed as a detector for LC.

4. In the above-mentioned Example 3, analysis of the peptide group is carried out by LC-MS/MS, but the disclosure is not limited thereby, and the Cleveland method, etc., may be utilized.

5. In the above-mentioned Example 1, at the time of extracting the protein, extraction is carried out without crushing the animal hair fibers, but the invention is not limited thereby, and the protein may be extracted after crushing the animal hair fibers. In particular, with regard to the descaled animal hair fibers, by finely crushing the fibers before extracting the protein, clear electrophoretic pattern can be easily obtained.

6. In the above-mentioned Example 2, an approximate expression by quadratic is used for the correction of the relative mobility Rf, but the invention is not limited thereby, and it may be the cubic equation or others when the correction can be carried out correctly.

7. In the above-mentioned Example 3, an approximate expression by quadratic is used for the correction of the retention time by LC-MS/MS, but the disclosure is not limited thereby, and it may be the cubic equation or others when the correction can be carried out correctly.

8. In the "checking test" in the above-mentioned Example 6, the electrophoretic pattern of the comparative sample is obtained in the same manner as in the above-mentioned first embodiment. Accordingly, consistency of the comparative sample and the sample to be tested is judged by comparing the electrophoretic patterns of the both samples. However, the disclosure is not limited thereby, and the mixing ratio of the comparative sample may be predicted from the database in the same manner as in the above-mentioned second embodiment. In this case, consistency of the mixing ratio of the comparative animal hair fibers obtained from the database, and the mixing ratio when the comparative animal hair fibers are prepared (which is the same as the mixing ratio of the animal hair fibers sample obtained from the database) is to be judged.

UTILIZABILITY IN INDUSTRY

A camouflage fiber product in which the other inexpensive animal hair fibers are mixed with the high class animal hair fibers such as cashmere, etc., have been on the market. In particular, in view of the current transactions of the fiber products being globalized, rapid and accurate discrimination method has been desired at the time of exporting and importing.

The present invention is to provide a precise discrimination means to the demand of the market, and it does not rely on experience of an inspector or know-how as in the conventional method. In particular, since camouflage is becoming more skillful in these days, the method that the kinds of the original animals of the animal hair fibers can be objectively discriminated, and even when camouflage has been done, accurate discrimination result can be obtained, is an epoch-making discrimination means.

From the facts as mentioned above, the present invention is to provide a discrimination means which is effective for stabilization of the market or international fair trade, which is not a discrimination means merely complementing the conventional methods JIS L 1030-1 (Testing methods for quantitative analysis of fibre mixtures-Part 1: Testing methods for fibre identification), and JIS L 1030-2 (Testing methods for quantitative analysis of fibre mixtures of textiles-Part 2: Testing methods for quantitative analysis of fibre mixtures), a discrimination means capable of utilizing as an international standard can be provided.

EXPLANATION OF REFERENCE SIGNS

A⋯Electrophoretic pattern of cashmere,
B⋯Electrophoretic pattern of camel,
C⋯Electrophoretic pattern of wool,
D⋯Electrophoretic pattern of yak,
E⋯Electrophoretic pattern of cashmere:yak=100%:0%,
F⋯Electrophoretic pattern of cashmere:yak=70%:30%,
G⋯Electrophoretic pattern of cashmere:yak=50%:50%,
H⋯Electrophoretic pattern of cashmere:yak=30%:70%,
I⋯Electrophoretic pattern of cashmere:yak=0%:100%,
J⋯Group of cashmere in database of electrophoretic patterns,
K⋯Group of other animal hair fibers in database of electrophoretic patterns,
L⋯Mass spectrometric pattern of cashmere,
M⋯Mass spectrometric pattern of camel,
N⋯Mass spectrometric pattern of wool,
O⋯Mass spectrometric pattern of yak,
P⋯Group of cashmere in database of mass spectrometric patterns,
Q⋯Group of mixed animal hair fibers of cashmere and yak in database of mass spectrometric patterns,

R···Group of other animal hair fibers in database of mass spectrometric patterns,
S···Electrophoretic pattern of animal hair fibers sample (cashmere),
T···Electrophoretic pattern of animal hair fibers sample (yak),
U···Electrophoretic pattern of animal hair fibers sample (mixture of cashmere and yak),
V···Mass spectrometric pattern of animal hair fibers sample (cashmere),
W···Mass spectrometric pattern of animal hair fibers sample (yak),
X···Mass spectrometric pattern of animal hair fibers sample (mixture of cashmere and yak),
Y···-Electrophoretic pattern of animal hair fibers sample (mixture),
Z···Electrophoretic pattern of standard sample (mixture of cashmere and wool),
AREA-1···Protein region specific to animal species,
AREA-2···Common protein region.

**Claims**

1. A discrimination method of animal hair fibers which comprises
   an extraction process for extracting a group of proteins from animal hair fibers which constitute the animal hair fibers sample,
   an electrophoretic process for obtaining an electrophoretic pattern by separating the extracted group of proteins with an electrophoretic means and staining the gel after the electrophoresis with a protein dyeing liquid, and
   a first discrimination process for discriminating kinds of animals serving as the origins of the animal hair fibers sample by comparing the obtained electrophoretic pattern with previously prepared electrophoretic patterns of single animal hair fibers in which kinds of animals serving as the origin have been known,
   wherein
   a series of electrophoretic patterns of previously prepared mixed animal hair fibers in which the animal hair fibers different in kinds of animals serving as the origin have been mixed with a series of mixing ratios is obtained in the same manner as in the extraction process and the electrophoretic process, and the method further comprises
   a second discrimination process for discriminating the animal hair fibers sample being originated from at least two kinds of animals, kinds of at least two kinds of animals serving as the origins of the animal hair fibers sample, and a mixing ratio of the animal hair fibers sample by comparing the electrophoretic pattern obtained from the animal hair fibers sample with a series of electrophoretic patterns obtained from the mixed animal hair fibers.

2. The discrimination method of animal hair fibers according to claim 1, wherein
   the method has a process for accumulating as a database by analyzing a relation of an intensity and relative mobility of each band with regard to the electrophoretic pattern of the single animal hair fibers, and a series of electrophoretic patterns obtained from the mixed animal hair fibers,
   a process for analyzing a relation between an intensity and a relative mobility of each band with regard to the electrophoretic patterns obtained from the animal hair fibers sample, and
   a third discrimination process for discriminating the animal hair fibers sample being originated from at least two kinds of animals, kinds of at least two kinds of animals serving as the origins of the animal hair fibers sample, and a mixing ratio of the animal hair fibers sample by collating analytical data of the animal hair fibers sample with a data group of the database, with consistency of the analytical data and the data group of the database as an index.

3. The discrimination method of animal hair fibers according to claim 1 or 2, wherein
   a mixing ratio of the animal hair fibers sample is discriminated by improving consistency of a mixing ratio of the animal hair fibers sample and a mixing ratio of the comparative animal hair fibers, by repeating at least once of each step of obtaining an electrophoretic pattern of comparative animal hair fibers in which using a mixing ratio of the animal hair fibers sample obtained in the second discrimination process or the third discrimination process, the single animal hair fibers in which the kinds of the original animals have been known are mixed with the same mixing ratio, and
   each step of carrying out discrimination of the second discrimination process or the third discrimination process again to the electrophoretic pattern of the obtained comparative animal hair fibers.

4. The discrimination method of animal hair fibers according to claim 1, wherein
   the mixed animal hair fibers are materials in which two kinds of the animal hair fibers in which the kinds of the original animals have been known are mixed with a series of mixing ratios, and the method further comprises
   a process for obtaining an intensity of a band at a previously recognized relative mobility which is specific to the two kinds of the animal hair fibers with regard to a series of electrophoretic pattern obtained from the mixed animal hair fibers,
a process for obtaining an intensity of a band at a previously recognized relative mobility with regard to the electrophoretic pattern obtained from the animal hair fibers sample, and
a fourth discrimination process for discriminating a mixing ratio of the animal hair fibers sample by collating the intensity of the band at the previously recognized relative mobility of the animal hair fibers sample with the intensity of the band at the previously recognized relative mobility of the mixed animal hair fibers, with consistency of intensities of these bands as an index.

5. The discrimination method of animal hair fibers according to claim 4, wherein
the two kinds of the animal hair fibers are cashmere and wool.

6. The discrimination method of animal hair fibers according to any one of claims 1 to 5, wherein
in the electrophoretic process for obtaining an electrophoretic pattern,
an electrophoretic gel having resolution at a region of a molecular weight of 25 kDa or less is capable of separating 10 or more bands is used at least to the electrophoretic pattern of the single animal hair fibers originating from cashmere among the electrophoretic patterns of the single animal hair fibers in which the kinds of the original animals have been known.

7. The discrimination method of animal hair fibers according to any one of claims 1 to 6, wherein
in the extraction process for extracting the group of proteins,
a reducing agent is used for splitting a disulfide bond in a molecule or between molecules of the protein, and the reducing agent is 2-mercaptoethanol with a concentration of more than 6% in a concentration of % by volume, or dithiothreitol with 8 to 12 mM (millimole/liter) in a molar concentration.

**Patentansprüche**

1. Verfahren zur Unterscheidung von Tierhaarfasern, umfassend
einen Extraktionsprozess zum Extrahieren einer Gruppe von Proteinen aus Tierhaarfasern, die die Tierhaarfaserprobe bilden,
einen Elektrophoreseprozess für den Erhalt eines Elektrophoresemusters durch Trennen der extrahierten Gruppe von Proteinen mit einem Elektrophoresemittel und Färben des Gels nach der Elektrophorese mit einer Proteinfärbeflüssigkeit, und
einen ersten Unterscheidungsprozess zum Unterscheiden von Tierarten, die als die Quelle der Tierhaarfaserprobe dienen, durch Vergleichen des erhaltenen Elektrophoresemusters mit zuvor hergestellten Elektrophoresemustern einzelner Tierhaarfasern, bei denen die Tierarten, die als die Quelle dienen, bekannt waren,
wobei
eine Reihe von Elektrophoresemustern zuvor hergestellter gemischter Tierhaarfasern, bei denen die Tierhaarfasern, die sich bei den Tierarten, die als die Quelle dienen, unterscheiden, mit einer Reihe von Mischverhältnissen gemischt wurden, auf dieselbe Weise erhalten wird wie in dem Extraktionsprozess und dem Elektrophoreseprozess und das Verfahren ferner umfasst
einen zweiten Unterscheidungsprozess zum Unterscheiden der Tierhaarfaserprobe, die von zumindest zwei Tierarten stammt, wobei die Arten zumindest zweier Tierarten als die Quellen der Tierhaarfaserprobe dienen, und des Mischverhältnisses der Tierhaarfaserprobe durch Vergleichen des Elektrophoresemusters, erhalten von der Tierhaarfaserprobe, mit einer Reihe von Elektrophoresemustern, erhalten von den gemischten Tierhaarfasern.

2. Verfahren zur Unterscheidung von Tierhaarfasern nach Anspruch 1, wobei
das Verfahren einen Prozess zum Datensammeln für eine Datenbank durch Analysieren einer Beziehung der Intensität und relativen Mobilität jeder Bande in Bezug auf das Elektrophoresemuster der einzelnen Tierhaarfasern und eine Reihe von Elektrophoresemustern, erhalten aus den gemischten Tierhaarfasern,
einen Prozess zum Analysieren einer Beziehung zwischen der Intensität und relativen Mobilität jeder Bande in Bezug auf die Elektrophoresemuster, erhalten aus der Tierhaarfaserprobe, und
einen dritten Unterscheidungsprozess zum Unterscheiden der Tierhaarfaserprobe, die von zumindest zwei Tierarten stammt, wobei die Arten zumindest zweier Tierarten als die Quellen der Tierhaarfaserprobe dienen, und des Mischverhältnisses der Tierhaarfaserprobe durch Vergleichen analytischer Daten der Tierhaarfaserprobe mit einer Datengruppe der Datenbank, wobei die Übereinstimmung der analytischen Daten und der Datengruppe der Datenbank als ein Index dient, aufweist.

**3.** Verfahren zur Unterscheidung von Tierhaarfasern nach Anspruch 1 oder 2, wobei das Mischverhältnis der Tierhaarfaserprobe durch Verbessern der Übereinstimmung des Mischverhältnisses der Tierhaarfaserprobe und des Mischverhältnisses der Vergleichstierhaarfasern unterschieden wird, wobei sowohl der Schritt des Erhaltens eines Elektrophoresemusters von Vergleichstierhaarfasern, bei dem, unter Verwendung eines Mischverhältnisses der Tierhaarfaserprobe, erhalten in dem zweiten Unterscheidungsprozess oder dem dritten Unterscheidungsprozess, die einzelnen Tierhaarfasern, wobei die Arten der ursprünglichen Tiere bekannt waren, mit demselben Mischverhältnis gemischt werden, als auch der Schritt des Durchführens der Unterscheidung des zweiten Unterscheidungsprozesses oder des dritten Unterscheidungsprozesses, erneut in Bezug auf das Elektrophoresemuster der erhaltenen Vergleichstierhaarfasern, zumindest einmal wiederholt wird.

**4.** Verfahren zur Unterscheidung von Tierhaarfasern nach Anspruch 1, wobei die gemischten Tierhaarfasern Materialien sind, bei denen die zwei Arten der Tierhaarfasern, wobei die Arten der ursprünglichen Tiere bekannt waren, mit einer Reihe von Mischverhältnissen gemischt werden, und das Verfahren ferner umfasst einen Prozess für den Erhalt der Intensität einer Bande bei einer zuvor erkannten relativen Mobilität, die für die zwei Arten der Tierhaarfasern spezifisch ist, in Bezug auf eine Reihe von Elektrophoresemustern, erhalten aus den gemischten Tierhaarfasern, einen Prozess für den Erhalt der Intensität einer Bande bei einer zuvor erkannten relativen Mobilität in Bezug auf das Elektrophoresemuster, erhalten aus der Tierhaarfaserprobe, und einen vierten Unterscheidungsprozess zum Unterscheiden eines Mischverhältnisses der Tierhaarfaserprobe durch Vergleichen der Intensität der Bande bei der zuvor erkannten relativen Mobilität der Tierhaarfaserprobe mit der Intensität der Bande bei der zuvor erkannten relativen Mobilität der gemischten Tierhaarfasern, wobei die Übereinstimmung der Intensitäten dieser Banden als ein Index dient.

**5.** Verfahren zur Unterscheidung von Tierhaarfasern nach Anspruch 4, wobei die zwei Arten von Tierhaarfasern Kaschmir und Wolle sind.

**6.** Verfahren zur Unterscheidung von Tierhaarfasern nach einem der Ansprüche 1 bis 5, wobei in dem Elektrophoreseprozess zum Erhalt eines Elektrophoresemusters ein Elektrophoresegel mit einem Auflösungsvermögen bei einer Molekulargewichtsregion von 25 kDa oder weniger, das 10 oder mehr Banden trennen kann, zumindest für das Elektrophoresemuster der einzelnen Tierhaarfasern, die von Kaschmir stammen, unter den Elektrophoresemustern der einzelnen Tierhaarfasern, wobei die Arten der ursprünglichen Tiere bekannt waren, verwendet wird.

**7.** Verfahren zur Unterscheidung von Tierhaarfasern nach einem der Ansprüche 1 bis 6, wobei in dem Extraktionsprozess zum Extrahieren der Gruppe von Proteinen ein Reduktionsmittel zum Aufspalten einer Disulfidbindung in einem Molekül oder zwischen Molekülen des Proteins verwendet wird und das Reduktionsmittel 2-Mercaptoethanol mit einer Konzentration von mehr als 6 % in einer Volumen-%-Konzentration oder Dithiothreitol mit 8 bis 12 mM (Millimol/Liter) in einer molaren Konzentration ist.


**Revendications**

**1.** Procédé de discrimination de fibres de poils d'animaux qui comprend un processus d'extraction pour extraire un groupe de protéines de fibres de poils d'animaux qui constituent l'échantillon de fibres de poils d'animaux, un processus électrophorétique pour obtenir un diagramme électrophorétique en séparant le groupe extrait de protéines avec un moyen électrophorétique et en colorant le gel après l'électrophorèse avec un liquide de teinture de protéine, et un premier processus de discrimination pour discriminer des sortes d'animaux servant d'origine de l'échantillon de fibres de poils d'animaux en comparant le diagramme électrophorétique obtenu à des diagrammes électrophorétiques préparés antérieurement de fibres de poils d'animaux uniques dans lesquelles des sortes d'animaux servant d'origine sont connues, dans lequel une série de diagrammes électrophorétiques de fibres de poils d'animaux mixtes préparées antérieurement dans lesquelles les fibres de poils d'animaux différant en termes de sortes d'animaux servant d'origine ont été mélangées à une série de rapports de mélange est obtenue de la même manière que dans le processus d'extraction et le processus électrophorétique, et le procédé comprend en outre
un deuxième processus de discrimination pour discriminer l'échantillon de fibres de poils d'animaux provenant d'au moins deux sortes d'animaux, des sortes d'au moins deux sortes d'animaux servant d'origine de l'échantillon de fibres de poils d'animaux, et un rapport de mélange de l'échantillon de fibres de poils d'animaux en comparant le diagramme électrophorétique obtenu à partir de l'échantillon de fibres de poils d'animaux à une série de diagrammes électrophorétiques obtenus à partir des fibres de poils d'animaux mixtes.

2. Procédé de discrimination de fibres de poils d'animaux selon la revendication 1, dans lequel
le procédé comporte un processus d'accumulation sous forme de base de données en analysant une relation d'une intensité et d'une mobilité relative de chaque bande par rapport au diagramme électrophorétique des fibres de poils d'animaux uniques, et une série de diagrammes électrophorétiques obtenus à partir des fibres de poils d'animaux mixtes,
un processus pour analyser une relation entre une intensité et une mobilité relative de chaque bande par rapport aux diagrammes électrophorétiques obtenus à partir de l'échantillon de fibres de poils d'animaux, et
un troisième processus de discrimination pour discriminer l'échantillon de fibres de poils d'animaux provenant d'au moins deux sortes d'animaux, des sortes d'au moins deux sortes d'animaux servant d'origine de l'échantillon de fibres de poils d'animaux, et un rapport de mélange de l'échantillon de fibres de poils d'animaux en rassemblant des données analytiques de l'échantillon de fibres de poils d'animaux avec un groupe de données de la base de données, avec la cohérence entre les données analytiques et le groupe de données de la base de données en tant qu'indice.

3. Procédé de discrimination de fibres de poils d'animaux selon la revendication 1 ou 2, dans lequel
un rapport de mélange de l'échantillon de fibres de poils d'animaux est discriminé en améliorant la cohérence entre un rapport de mélange de l'échantillon de fibres de poils d'animaux et un rapport de mélange des fibres de poils d'animaux comparatives, en répétant au moins une fois l'une parmi
chaque étape d'obtention d'un diagramme électrophorétique de fibres de poils d'animaux comparatives dans laquelle en utilisant un rapport de mélange de l'échantillon de fibres de poils d'animaux obtenu dans le deuxième processus de discrimination ou le troisième processus de discrimination, les fibres de poils d'animaux uniques dans lesquelles les sortes des animaux d'origine sont connues sont mélangées avec le même rapport de mélange, et
chaque étape de réalisation d'une discrimination du deuxième processus de discrimination ou du troisième processus de discrimination à nouveau pour le diagramme électrophorétique des fibres de poils d'animaux comparatives obtenues.

4. Procédé de discrimination de fibres de poils d'animaux selon la revendication 1, dans lequel
les fibres de poils d'animaux mixtes sont des matériaux dans lesquels deux sortes des fibres de poils d'animaux dans lesquelles les sortes des animaux d'origine sont connues sont mélangées avec une série de rapports de mélange, et le procédé comprend en outre
un processus pour obtenir une intensité d'une bande à une mobilité relative reconnue antérieurement qui est spécifique des deux sortes des fibres de poils d'animaux par rapport à une série de diagrammes électrophorétiques obtenus à partir des fibres de poils d'animaux mixtes,
un processus pour obtenir une intensité d'une bande à une mobilité relative reconnue antérieurement par rapport au diagramme électrophorétique obtenu à partir de l'échantillon de fibres de poils d'animaux, et
un quatrième processus de discrimination pour discriminer un rapport de mélange de l'échantillon de fibres de poils d'animaux en rassemblant l'intensité de la bande à la mobilité relative reconnue antérieurement de l'échantillon de fibres de poils d'animaux avec l'intensité de la bande à la mobilité relative reconnue antérieurement des fibres de poils d'animaux mixtes, avec la cohérence entre les intensités de ces bandes comme indice.

5. Procédé de discrimination de fibres de poils d'animaux selon la revendication 4, dans lequel
les deux sortes des fibres de poils d'animaux sont le cachemire et la laine.

6. Procédé de discrimination de fibres de poils d'animaux selon l'une quelconque des revendications 1 à 5, dans lequel
dans le processus électrophorétique pour obtenir un diagramme électrophorétique,
un gel électrophorétique ayant une résolution au niveau d'une région de masse moléculaire de 25 kDa ou moins capable de séparer 10 bandes ou plus est utilisé au moins pour le diagramme électrophorétique des fibres de poils d'animaux uniques provenant du cachemire parmi les diagrammes électrophorétiques des fibres de poils d'animaux uniques dans lesquelles les sortes des animaux d'origine sont connues.

7. Procédé de discrimination de fibres de poils d'animaux selon l'une quelconque des revendications 1 à 6, dans lequel

dans le processus d'extraction pour extraire le groupe de protéines,
un agent réducteur est utilisé pour scinder une liaison disulfure dans une molécule ou entre des molécules de la protéine, et l'agent réducteur est le 2-mercaptoéthanol de concentration supérieure à 6 % dans une concentration en % en volume, ou le dithiothréitol dans une concentration molaire de 8 à 12 mM (millimole/litre).

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

**REFERENCES CITED IN THE DESCRIPTION**

**Patent documents cited in the description**

- JP 2000210084 A
- JP 2004121229 A

- EP 1847831 A1

**Non-patent literature cited in the description**

- **MARSHALL RC et al.** *Journal of the Forensic Science Society,* 1985, vol. 25, 57-66